# EUROPEAN PATENT SPECIFICATION

(11) **EP 4 196 257 B1**
(45) Date of publication and mention of the grant of the patent: **03.06.2026**
(21) Application number: 21823617.2
(22) Date of filing: 13.12.2021
(51) Int. Cl.: B01J 13/16, A01N 25/28, A23P 10/30, A61K 8/11, A61K 9/50, C11D 3/50

(54) **PROCESS FOR PREPARING POLYESTER MICROCAPSULES**
VERFAHREN ZUR HERSTELLUNG VON POLYESTERMIKROKAPSELN
PROCÉDÉ DE PRÉPARATION DE MICROCAPSULES DE POLYESTER

(30) Priority: 21.12.2020 EP 20216043
(43) Date of publication of application: 21.06.2023
(73) Proprietor: Firmenich SA, 1242 Satigny (CH)
(72) Inventor: LEON, Geraldine, 1242 Satigny (CH); BERTHIER, Damien, 1242 Satigny (CH); OUALI, Lahoussine, 1242 Satigny (CH); ERNI, Philipp, 1242 Satigny (CH); ELABBADI, Amal, 1242 Satigny (CH); BEAUSSOUBRE, Pascal, 1242 Satigny (CH)
(74) Representative: Strych, Sebastian
(86) International application number: PCT/EP2021/085534
(87) International publication number: WO 2022/136008

(56) References cited:
- WO-A1-2014/187833
- WO-A1-2014/187874
- WO-A1-2016/083321
- WO-A1-2019/002380
- WO-A1-2021/023647
- WO-A2-2013/182855
- KR-A- 20200 004 152
- KR-A- 20200 004 153
- US-A1- 2005 106 232
- US-A1- 2012 129 742
- JIN C ET AL: "Preparation of organic/inorganic hybrid microcapsules involves performing Pickering emulsion by adding dispersed phase solution comprising polymer precursor to continuous phase solution comprising inorganic nanoparticles, and polymerizing", WPI / 2017 CLARIVATE ANALYTICS,, vol. 2020, no. 9, 9 January 2020 (2020-01-09), XP002803128
- DATABASE WPI Week 2020, Derwent World Patents Index; AN 2020-04507F, XP002803128

## Description

### Technical Field

The present invention relates to a new process for the preparation of polyester microcapsules. Microcapsules are also an object of the invention as well as consumer products comprising said microcapsules, in the form of a personal care, a home care or a fabric care composition.

### Background of the Invention

One of the problems faced by the perfume and flavour industry lies in the relatively rapid loss of olfactive benefit provided by active compounds due to their volatility. The encapsulation of those active substances provides at the same time a protection of the ingredients there-encapsulated against "aggressions" such as oxidation or moisture and allows, on the other hand, a certain control of the kinetics of flavour or fragrance release to induce sensory effects through sequential release.

Polyurea and polyurethane-based microcapsule slurry are widely used for example in perfumery industry for instance as they provide a long lasting pleasant olfactory effect after their applications on different substrates. Those microcapsules have been widely disclosed in the prior art (see for example WO2007/004166 or EP 2300146 from the Applicant).

US 2005/106232 A1 discloses a method of preparing composite capsules by means of interfacial polycondensation in a dispersed medium of at least two different monomers.

In addition to the performance in terms of stability and olfactive performance, the consumer demand for eco-friendly delivery systems is more and more important and is driving the development of new delivery systems.

There is therefore still a need to provide new microcapsules using more eco-friendly materials, while not compromising on the performance of the microcapsules, in particular in terms of stability in a challenging medium such as a consumer product base, as well as in delivering a good performance in terms of active ingredient delivery, e.g. olfactive performance in the case of perfuming ingredients.

The present invention is proposing a solution to the above-mentioned problem, based on polyester microcapsules obtained from polyol-based multiple emulsions.

### Detailed description of the Invention

Unless stated otherwise, percentages (%) are meant to designate a percentage by weight of a composition.

By **"hydrophobic material",** it is meant any hydrophobic material - single material or a mixture of material - which forms a two-phase dispersion when mixed with water.

By **"ingredient",** it is meant a single compound or a combination of ingredients.

By **"perfume or flavor oil",** it is meant a single perfuming or flavoring compound or a mixture of several perfuming or flavoring compounds.

By **"consumer product" or "end-product"** it is meant a manufactured product ready to be distributed, sold and used by a consumer.

By **"polyester microcapsule"** it is meant that the microcapsule comprises a polymer containing ester linkages by a polyol capable of further reacting with a carboxylic acid derivative, for example with an acyl chloride.

By **"polyol",** it is meant a compound having at least two hydroxyl functions.

By **"carboxylic acid derivative",** it means a compound with an acyl group bounded to an electronegative atom or substituent, -X, that can act as a leaving group in substitution reactions.

Electronegative atom may be alkyl amine, alkyl alcohol, amine or hydroxyl, group, and -X may be chlorides, bromides, tosylates, mesylates, carboxylic acids (as anhydrides).

For the sake of clarity, by the expression **"dispersion"** in the present invention it is meant a system in which particles and/or oil droplets are dispersed in a continuous phase of a different composition and it specifically includes a suspension or an emulsion.

By **"multiple dispersion",** it means oil-in-polyol-in-oil multiple emulsion.

By **"carrier"** or **"carrier material"** is herein understood that the material of the carrier (i.e polyester carrier) is suitable to entrap, encapsulate or hold a certain amount of hydrophobic material.

Typically, when microcapsule is in a matrix form, the carrier material is a matrix material and the microcapsule has to entrap preferably at least 20 wt.%, preferably at least 30 wt.%, even more preferably at least 35 wt.% of the hydrophobic material, based on the total weight of the microcapsule.

Typically, when the microcapsule is in the form of a core-shell microcapsule, the carrier is a shell and the microcapsule has to entrap preferably at least 80 wt.%, preferably at least 90 wt.%, of the hydrophobic material, based on the total weight of the microcapsule.

In a particular embodiment, the carrier or carrier material is a solid carrier material, i.e. an emulsion or solvent is not a carrier or carrier material.

A **"microcapsule",** or the similar, in the present invention it is meant that capsules have a particle size distribution in the micron range (e.g. a mean diameter (d(v, 0.5)) preferably comprised between about 1 and 3000 microns, preferably between 1 and 500 microns) According to the invention, the wordings "mean diameter" or "mean size" are used indifferently.

By **"microcapsule slurry",** it is meant microcapsule(s) that is (are) dispersed in a liquid. According to an embodiment, the microcapsule(s) is (are) dispersed in a hydrophilic phase.

By" **a carboxylic acid derivative",** it should be understood a single carboxylic acid derivative or a mixture of carboxylic acid derivatives.

It has been found that polyester microcapsules could be obtained based on the preparation of hydrophobic material-in-polyol-in-oil multiple emulsion and the reaction between carboxylic acid derivative and polyol.

A first object of the invention is therefore a process for preparing a polyester microcapsule slurry, said process comprising the steps of:
(i) Dispersing an oil phase O1 comprising a hydrophobic material H1, into a continuous phase comprising a polyol to obtain a two-phases dispersion E1, wherein the oil phase O1 and/or the continuous phase comprise a stabilizer S1,
(ii) Dispersing the two-phases dispersion E1 in an oil phase O2 comprising a hydrophobic material H2 and a stabilizer S2 to obtain a multiple dispersion E2;
(iii) Adding at least one carboxylic acid derivative A3 into the multiple dispersion E2; and
(iv) applying conditions sufficient to induce interfacial polymerization and form polyester microcapsules in the form of a slurry, wherein the carboxylic acid derivative A3 is chosen in the group consisting of acid, amide, ester, anhydride and acid chloride and mixtures thereof.
In a step of the process of the invention, an oil phase O1 comprising a hydrophobic material H1 is dispersed into a continuous phase comprising a polyol to obtain a two-phases dispersion E1, wherein the oil phase O1 and/or the continuous phase comprise a stabilizer S1

### Oil phase O1

According to the invention, the oil phase O1 comprises a hydrophobic material H1.

According to a particular embodiment, the oil phase O1 consists of a hydrophobic material H1.

The hydrophobic material according to the invention can be "inert" material like solvents or active ingredients. A single hydrophobic material or mixture of hydrophobic materials can be used.

When the hydrophobic material is an active ingredient, it is preferably chosen from the group consisting of flavor, flavor ingredients, perfume, perfume ingredients, nutraceuticals, cosmetics, pest control agents, biocide actives, malodour counteracting ingredient, bactericide ingredient, fungicide ingredient, pharmaceutical or agrochemical ingredient, a sanitizing ingredient, an insect repellent or attractant, and mixtures thereof.

According to a particular embodiment, the hydrophobic material comprises a phase change material (PCM).

According to a particular embodiment, the hydrophobic material comprises a mixture of a perfume with another ingredient selected from the group consisting of nutraceuticals, cosmetics, pest control agents and biocide actives.

According to a particular embodiment, the hydrophobic material comprises a mixture of biocide actives with another ingredient selected from the group consisting of perfume, nutraceuticals, cosmetics, pest control agents.

According to a particular embodiment, the hydrophobic material comprises a mixture of pest control agents with another ingredient selected from the group consisting of perfume, nutraceuticals, cosmetics, biocide actives.

According to a particular embodiment, the hydrophobic material comprises a perfume.

According to a particular embodiment, the hydrophobic material consists of a perfume.

According to a particular embodiment, the hydrophobic material consists of biocide actives.

According to a particular embodiment, the hydrophobic material consists of pest control agents.

By "perfume" (or also "perfume oil") what is meant here is an ingredient or a composition that is a liquid at about 20°C. According to any one of the above embodiments said perfume oil can be a perfuming ingredient alone or a mixture of ingredients in the form of a perfuming composition. As a "perfuming ingredient" it is meant here a compound, which is used for the primary purpose of conferring or modulating an odor. In other words such an ingredient, to be considered as being a perfuming one, must be recognized by a person skilled in the art as being able to at least impart or modify in a positive or pleasant way the odor of a composition, and not just as having an odor. For the purpose of the present invention, perfume oil also includes a combination of perfuming ingredients with substances which together improve, enhance or modify the delivery of the perfuming ingredients, such as perfume precursors, modulators, emulsions or dispersions, as well as combinations which impart an additional benefit beyond that of modifying or imparting an odor, such as long-lastingness, blooming, malodor counteraction, antimicrobial effect, microbial stability, pest control.

The nature and type of the perfuming ingredients present in the oil phase do not warrant a more detailed description here, which in any case would not be exhaustive, the skilled person being able to select them on the basis of its general knowledge and according to intended use or application and the desired organoleptic effect. In general terms, these perfuming ingredients belong to chemical classes as varied as alcohols, aldehydes, ketones, esters, ethers, acetates, nitriles, terpenoids, nitrogenous or sulfurous heterocyclic compounds and essential oils (for example thyme oil), and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery.

In particular one may cite perfuming ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0^{2,7}]undecan-4-one, 1-methoxy-3-hexanethiol, 2-ethyl-4,4-dimethyl-1,3-oxathiane, 2,2,7/8,9/10-tetramethylspiro[5.5]undec-8-en-1-one, menthol and/or alpha-pinene;

- Balsamic ingredients: coumarin, ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
   - Floral ingredients: methyl dihydrojasmonate, linalool, citronellol, phenylethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, 1-(2,6,6-trimethyl-1,3-cyclohexadien-1-yl)-2-buten-1-one, (2E)-1-(2,6,6-trimethyl-2-cyclohexen-1-yl)-2-buten-1-one, (2E)-1-[2,6,6-trimethyl-3-cyclohexen-1-yl]-2-buten-1-one, (2E)-1-(2,6,6-trimethyl-1-cyclohexen-1-yl)-2-buten-1-one, 3-(3,3/1,1-dimethyl-5-indanyl)propanal, 2,5-dimethyl-2-indanmethanol, 2,6,6-trimethyl-3-cyclohexene-1-carboxylate, 3-(4,4-dimethyl-1-cyclohexen-1-yl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 8-decen-5-olide, 4-phenyl-2-butanone, isononyle acetate, 4-(1,1-dimethylethyl)-1-cyclohexyl acetate, verdyl isobutyrate and/or mixture of methylionones isomers;
   - Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
- Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
- Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{(1S)-1-[(1R)-3,3-dimethylcyclohexyl]ethoxy}-2-oxoethyl propionate, 3-methyl-5-cyclopentadecen-1-one, 4,6,6,7,8,8-hexamethyl-1,3,4,6,7,8-hexahydrocyclopenta[g]isochromene, (1S,1'R)-2-[1-(3',3'-dimethyl-1'-cyclohexyl)ethoxy]-2-methylpropyl propanoate, oxacyclohexadecan-2-one and/or (1S,1'R)-[1-(3',3'-dimethyl-1'-cyclohexyl)ethoxycarbonyl]methyl propanoate;
- Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2.1.0^{2,7}]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, Clearwood^{®}, (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3-methylpentan-2-ol, 1-(2,3,8,8-tetramethyl-1,2,3,4,6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonane and/or 3-(3-isopropyl-1-phenyl)butanal.
It is also understood that said ingredients may also be compounds known to release in a controlled manner various types of perfuming compounds also known as properfume or profragrance. Non-limiting examples of suitable properfumes may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, 3-(dodecylthio)-1-(2,6,6-trimethyl-3-cyclohexen-1-yl)-1-butanone, 2-(dodecylthio)octan-4-one, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1-(((Z)-hex-3-en-1-yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, 2-(1-phenethoxyprop-1-en-2-yl)naphthalene, (2-phenethoxyvinyl)benzene, 2-(1-((3,7-dimethyloct-6-en-1-yl)oxy)prop-1-en-2-yl)naphthalene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 3-methoxy-4-((2-methoxy-2-phenylvinyl)oxy)benzaldehyde, 4-((2-(hexyloxy)-2-phenylvinyl)oxy)-3-methoxybenzaldehyde or a mixture thereof.

The perfuming ingredients may be dissolved in a solvent of current use in the perfume industry. The solvent is preferably not an alcohol. Examples of such solvents are diethyl phthalate, isopropyl myristate, Abalyn^{®} (rosin resins, available from Eastman), benzyl benzoate, ethyl citrate, triethyl citrate, limonene or other terpenes, or isoparaffins. Preferably, the solvent is very hydrophobic and highly sterically hindered, like for example Abalyn^{®} or benzyl benzoate. Preferably the perfume comprises less than 30% of solvent. More preferably the perfume comprises less than 20% and even more preferably less than 10% of solvent, all these percentages being defined by weight relative to the total weight of the perfume. Most preferably, the perfume is essentially free of solvent.

Preferred perfuming ingredients are those having a high steric hindrance (bulky materials) and in particular those from one of the following groups:
- Group 1: perfuming ingredients comprising a cyclohexane, cyclohexene, cyclohexanone or cyclohexenone ring substituted with at least one linear or branched C₁ to C₄ alkyl or alkenyl substituent;
- Group 2: perfuming ingredients comprising a cyclopentane, cyclopentene, cyclopentanone or cyclopentenone ring substituted with at least one linear or branched C₄ to C₈ alkyl or alkenyl substituent;
- Group 3: perfuming ingredients comprising a phenyl ring or perfuming ingredients comprising a cyclohexane, cyclohexene, cyclohexanone or cyclohexenone ring substituted with at least one linear or branched C₅ to C₈ alkyl or alkenyl substituent or with at least one phenyl substituent and optionally one or more linear or branched C₁ to C₃ alkyl or alkenyl substituents;
- Group 4: perfuming ingredients comprising at least two fused or linked C₅ and/or C₆ rings;
- Group 5: perfuming ingredients comprising a camphor-like ring structure;
- Group 6: perfuming ingredients comprising at least one C₇ to C₂₀ ring structure;
- Group 7: perfuming ingredients having a logP value above 3.5 and comprising at least one tert-butyl or at least one trichloromethyl substitutent;

Examples of ingredients from each of these groups are:
- Group 1: 2,4-dimethyl-3-cyclohexene-1-carbaldehyde (origin: Firmenich SA, Geneva, Switzerland), isocyclocitral, menthone, isomenthone, methyl 2,2-dimethyl-6-methylene-1-cyclohexanecarboxylate (origin: Firmenich SA, Geneva, Switzerland), nerone, terpineol, dihydroterpineol, terpenyl acetate, dihydroterpenyl acetate, dipentene, eucalyptol, hexylate, rose oxide, (S)-1,8-p-menthadiene-7-ol (origin: Firmenich SA, Geneva, Switzerland), 1-p-menthene-4-ol, (1RS,3RS,4SR)-3-p-mentanyl acetate, (1R,2S,4R)-4,6,6-trimethyl-bicyclo[3,1,1]heptan-2-ol, tetrahydro-4-methyl-2-phenyl-2H-pyran (origin: Firmenich SA, Geneva, Switzerland), cyclohexyl acetate, cyclanol acetate, 1,4-cyclohexane diethyldicarboxylate (origin: Firmenich SA, Geneva, Switzerland), (3RS,3aRS,6SR,7ASR)-perhydro-3,6-dimethyl-benzo[B]furan-2-one (origin: Firmenich SA, Geneva, Switzerland), ((6R)-perhydro-3,6-dimethyl-benzo[B]furan-2-one (origin: Firmenich SA, Geneva, Switzerland), 2,4,6-trimethyl-4-phenyl-1,3-dioxane, 2,4,6-trimethyl-3-cyclohexene-1-carbaldehyde;
- Group 2: (E)-3-methyl-5-(2,2,3-trimethyl-3-cyclopenten-1-yl)-4-penten-2-ol (origin: Givaudan SA, Vernier, Switzerland), (1'R,E)-2-ethyl-4-(2',2',3'-trimethyl-3'-cyclopenten-1'-yl)-2-buten-1-ol (origin: Firmenich SA, Geneva, Switzerland), (1'R,E)-3,3-dimethyl-5-(2',2',3'-trimethyl-3'-cyclopenten-1'-yl)-4-penten-2-ol (origin: Firmenich SA, Geneva, Switzerland), 2-heptylcyclopentanone, methyl-cis-3-oxo-2-pentyl-1-cyclopentane acetate (origin: Firmenich SA, Geneva, Switzerland), 2,2,5-trimethyl-5-pentyl-1-cyclopentanone (origin: Firmenich SA, Geneva, Switzerland), 3,3-dimethyl-5-(2,2,3-trimethyl-3-cyclopenten-1-yl)-4-penten-2-ol (origin: Firmenich SA, Geneva, Switzerland), 3-methyl-5-(2,2,3-trimethyl-3-cyclopenten-1-yl)-2-pentanol (origin, Givaudan SA, Vernier, Switzerland);
- Group 3: damascones, 1-(5,5-dimethyl-1-cyclohexen-1-yl)-4-penten-1-one (origin: Firmenich SA, Geneva, Switzerland), (1'R)-2-[2-(4'-methyl-3'-cyclohexen-1'-yl)propyl]cyclopentanone, alpha-ionone, beta-ionone, damascenone, mixture of 1-(5,5-dimethyl-1-cyclohexen-1-yl)-4-penten-1-one and 1-(3,3-dimethyl-1-cyclohexen-1-yl)-4-penten-1-one (origin: Firmenich SA, Geneva, Switzerland), 1-(2,6,6-trimethyl-1-cyclohexen-1-yl)-2-buten-1-one (origin: Firmenich SA, Geneva, Switzerland), (15,1'R)-[1-(3',3'-Dimethyl-1'-cyclohexyl)ethoxycarbonyl]methyl propanoate (origin: Firmenich SA, Geneva, Switzerland), 2-tert-butyl-1-cyclohexyl acetate (origin: International Flavors and Fragrances, USA), 1-(2,2,3,6-tetramethyl-cyclohexyl)-3-hexanol (origin: Firmenich SA, Geneva, Switzerland), trans-1-(2,2,6-trimethyl-1-cyclohexyl)-3-hexanol (origin: Firmenich SA, Geneva, Switzerland), (E)-3-methyl-4-(2,6,6-trimethyl-2-cyclohexen-1-yl)-3-buten-2-one, terpenyl isobutyrate, 4-(1,1-dimethylethyl)-1-cyclohexyl acetate (origin: Firmenich SA, Geneva, Switzerland), 8-methoxy-1-p-menthene, (1S,1'R)-2-[1-(3',3'-dimethyl-1'-cyclohexyl) ethoxy]-2-methylpropyl propanoate (origin: Firmenich SA, Geneva, Switzerland), para tert-butylcyclohexanone, menthenethiol, 1-methyl-4-(4-methyl-3-pentenyl)-3-cyclohexene-1-carbaldehyde, allyl cyclohexylpropionate, cyclohexyl salicylate, 2-methoxy-4-methylphenyl methyl carbonate, ethyl 2-methoxy-4-methylphenyl carbonate, 4-ethyl-2-methoxyphenyl methyl carbonate;
- Group 4: Methyl cedryl ketone (origin: International Flavors and Fragrances, USA), a mixture of (1RS,2SR,6RS,7RS,8SR)-tricyclo[5.2.1.0^{2,6}]dec-3-en-8-yl 2-methylpropanoate and (1RS,2SR,6RS,7RS,8SR)-tricyclo[5.2.1.0^{2,6}]dec-4-en-8-yl 2-methylpropanoate, vetyverol, vetyverone, 1-(octahydro-2,3,8,8-tetramethyl-2-naphtalenyl)-1-ethanone (origin: International Flavors and Fragrances, USA), (5RS,9RS,10SR)-2,6,9,10-tetramethyl-1-oxaspiro[4.5]deca-3,6-diene and the (5RS,9SR,10RS) isomer, 6-ethyl-2,10,10-trimethyl-1-oxaspiro[4.5]deca-3,6-diene, 1,2,3,5,6,7-hexahydro-1,1,2,3,3-pentamethyl-4-indenone (origin: International Flavors and Fragrances, USA), a mixture of 3-(3,3-dimethyl-5-indanyl)propanal and 3-(1,1-dimethyl-5-indanyl)propanal (origin: Firmenich SA, Geneva, Switzerland), 3',4-dimethyl-tricyclo[6.2.1.0(2,7)]undec-4-ene-9-spiro-2'-oxirane (origin: Firmenich SA, Geneva, Switzerland), 9/10-ethyldiene-3-oxatricyclo[6.2.1.0(2,7)]undecane, (perhydro-5,5,8A-trimethyl-2-naphthalenyl acetate (origin: Firmenich SA, Geneva, Switzerland), octalynol, (dodecahydro-3a,6,6,9a-tetramethyl-naphtho[2,1-b]furan, origin: Firmenich SA, Geneva, Switzerland), tricyclo[5.2.1.0(2,6)]dec-3-en-8-yl acetate and tricyclo[5.2.1.0(2,6)]dec-4-en-8-yl acetate as well as tricyclo[5.2.1.0(2,6)]dec-3-en-8-yl propanoate and tricyclo[5.2.1.0(2,6)]dec-4-en-8-yl propanoate, (+)-(1S,2S,3S)-2,6,6-trimethyl-bicyclo[3.1.1]heptane-3-spiro-2'-cyclohexen-4'-one;
- Group 5: camphor, borneol, isobornyl acetate, 8-isopropyl-6-methyl-bicyclo[2.2.2]oct-5-ene-2-carbaldehyde, pinene, camphene, 8-methoxycedrane, (8-methoxy-2,6,6,8-tetramethyl-tricyclo[5.3.1.0(1,5)]undecane (origin: Firmenich SA, Geneva, Switzerland), cedrene, cedrenol, cedrol, mixture of 9-ethylidene-3-oxatricyclo[6.2.1.0(2,7)]undecan-4-one and 10-ethylidene-3-oxatricyclo[6.2.1.0^{2,7}]undecan-4-one (origin: Firmenich SA, Geneva, Switzerland), 3-methoxy-7,7-dimethyl-10-methylene-bicyclo[4.3.1]decane (origin: Firmenich SA, Geneva, Switzerland);
- Group 6: (trimethyl-13-oxabicyclo-[10.1.0]-trideca-4,8-diene (origin: Firmenich SA, Geneva, Switzerland), 9-hexadecen-16-olide (origin: Firmenich SA, Geneva, Switzerland), pentadecenolide (origin: Firmenich SA, Geneva, Switzerland), 3-methyl-(4/5)-cyclopentadecenone ,(origin: Firmenich SA, Geneva, Switzerland), 3-methylcyclopentadecanone (origin: Firmenich SA, Geneva, Switzerland), pentadecanolide (origin: Firmenich SA, Geneva, Switzerland), cyclopentadecanone (origin: Firmenich SA, Geneva, Switzerland), 1-ethoxyethoxy)cyclododecane (origin: Firmenich SA, Geneva, Switzerland), 1,4-dioxacycloheptadecane-5,17-dione, 4,8-cyclododecadien-1-one;
- Group 7: (+-)-2-methyl-3-[4-(2-methyl-2-propanyl)phenyl]propanal (origin: Givaudan SA, Vernier, Switzerland), 2,2,2-trichloro-1-phenylethyl acetate.

Preferably, the perfume comprises at least 30%, preferably at least 50%, more preferably at least 60% of ingredients selected from Groups 1 to 7, as defined above. More preferably said perfume comprises at least 30%, preferably at least 50% of ingredients from Groups 3 to 7, as defined above. Most preferably said perfume comprises at least 30%, preferably at least 50% of ingredients from Groups 3, 4, 6 or 7, as defined above.

According to another preferred embodiment, the perfume comprises at least 30%, preferably at least 50%, more preferably at least 60% of ingredients having a logP above 3, preferably above 3.5 and even more preferably above 3.75.

According to a particular embodiment, the perfume used in the invention contains less than 10% of its own weight of primary alcohols, less than 15% of its own weight of secondary alcohols and less than 20% of its own weight of tertiary alcohols. Advantageously, the perfume used in the invention does not contain any primary alcohols and contains less than 15% of secondary and tertiary alcohols.

According to an embodiment, the oil phase (or the oil-based core) comprises:
- 25-100wt% of a perfume oil comprising at least 15wt% of high impact perfume raw materials having a Log T<-4, and
- 0-75wt% of a density balancing material having a density greater than 1.07 g/cm³.
*"High impact perfume raw materials"* should be understood as perfume raw materials having a LogT<-4. The odor threshold concentration of a chemical compound is determined in part by its shape, polarity, partial charges and molecular mass. For convenience, the odor threshold concentration is presented as the common logarithm of the threshold concentration, i.e., Log [Threshold] ("LogT").

*A "density balancing material"* should be understood as a material having a density greater than 1.07 g/cm³ and having preferably low or no odor.
The odor threshold concentration of a perfuming compound is determined by using a gas chromatograph ("GC"). Specifically, the gas chromatograph is calibrated to determine the exact volume of the perfume oil ingredient injected by the syringe, the precise split ratio, and the hydrocarbon response using a hydrocarbon standard of known concentration and chain-length distribution. The air flow rate is accurately measured and, assuming the duration of a human inhalation to last 12 seconds, the sampled volume is calculated. Since the precise concentration at the detector at any point in time is known, the mass per volume inhaled is known and hence the concentration of the perfuming compound. To determine the threshold concentration, solutions are delivered to the sniff port at the back-calculated concentration. A panelist sniffs the GC effluent and identifies the retention time when odor is noticed. The average across all panelists determines the odor threshold concentration of the perfuming compound. The determination of odor threshold is described in more detail in C. Vuilleumier et al., Multidimensional Visualization of Physical and Perceptual Data Leading to a Creative Approach in Fragrance Development, Perfume & Flavorist, Vol. 33, September,, 2008, pages 54-61.

The nature of high impact perfume raw materials having a Log T<-4 and density balancing material having a density greater than 1.07 g/cm³ are described in WO2018115250.

According to an embodiment, the high impact perfume raw materials having a Log T<-4 are selected from the group consisting of (+-)-1-methoxy-3-hexanethiol, 4-(4-hydroxy-1-phenyl)-2-butanone, 2-methoxy-4-(1-propenyl)-1-phenyl acetate, pyrazobutyle, 3-propylphenol, 1-(3-methyl-1-benzofuran-2-yl)ethanone, 2-(3-phenylpropyl)pyridine, 1-(3,3/5,5-dimethyl-1-cyclohexen-1-yl)-4-penten-1-one , 1-(5,5-dimethyl-1-cyclohexen-1-yl)-4-penten-1-one, a mixture comprising (3RS,3aRS,6SR,7ASR)-perhydro-3,6-dimethyl-benzo[b]furan-2-one and (3SR,3aRS,6SR,7ASR)-perhydro-3,6-dimethyl-benzo[b]furan-2-one, (+-)-1-(5-ethyl-5-methyl-1-cyclohexen-1-yl)-4-penten-1-one, (1'S,3'R)-1-methyl-2-[(1',2',2'-trimethylbicyclo[3.1.0]hex-3'-yl)methyl]cyclopropyl}methanol, (+-)-3-mercaptohexyl acetate, (2E)-1-(2,6,6-trimethyl-1,3-cyclohexadien-1-yl)-2-buten-1-one, H-methyl-2h-1,5-benzodioxepin-3(4H)-one, (2E,6Z)-2,6-nonadien-1-ol, (4Z)-4-dodecenal, (+-)-4-hydroxy-2,5-dimethyl-3(2H)-furanone, methyl 2,4-dihydroxy-3,6-dimethylbenzoate, 3-methylindole, (+-)-perhydro-4alpha,8abeta-dimethyl-4a-naphthalenol, patchoulol, 2-methoxy-4-(1-propenyl)phenol, mixture comprising (+-)-5,6-dihydro-4-methyl-2-phenyl-2H-pyran and tetrahydro-4-methylene-2-phenyl-2H-pyran, mixture comprising 4-methylene-2-phenyltetrahydro-2H-pyran and (+-)-4-methyl-2-phenyl-3,6-dihydro-2H-pyran, 4-hydroxy-3-methoxybenzaldehyde, nonylenic aldehyde, 2-methoxy-4-propylphenol, 3-methyl-5-phenyl-2-pentenenitrile, 1-(spiro[4.5]dec-6/7-en-7-yl)-4-penten-1-one(, 2-methoxynaphthalene, (-)-(3aR,5AS,9AS,9BR)-3a,6,6,9a-tetramethyldodecahydronaphtho[2,1-b]furan, 5-nonanolide, (3aR,5AS,9AS,9BR)-3a,6,6,9a-tetramethyldodecahydronaphtho[2,1-b]furan, 7-isopropyl-2H,4H-1,5-benzodioxepin-3-one, coumarin, 4-methylphenyl isobutyrate, (2E)-1-(2,6,6-trimethyl-1,3-cyclohexadien-1-yl)-2-buten-1-one, beta,2,2,3-tetramethyl-delta-methylene-3-cyclopentene-1-butanol, delta damascone ((2E)-1-[(1RS,2SR)-2,6,6-trimethyl-3-cyclohexen-1-yl]-2-buten-1-one), (+-)-3,6-dihydro-4,6-dimethyl-2-phenyl-2h-pyran, anisaldehyde, paracresol, 3-ethoxy-4-hydroxybenzaldehyde, methyl 2-aminobenzoate, ethyl methylphenylglycidate, octalactone gamma, ethyl 3-phenyl-2-propenoate, (-)-(2E)-2-ethyl-4-[(1R)-2,2,3-trimethyl-3-cyclopenten-1-yl]-2-buten-1-ol, paracresyl acetate, dodecalactone, tricyclone, (+)-(3R,5Z)-3-methyl-5-cyclopentadecen-1-one, undecalactone, (1R,4R)-8-mercapto-3-p-menthanone, (3S,3AS,6R,7AR)-3,6-dimethylhexahydro-1-benzofuran-2(3H)-one, beta ionone, (+-)-6-pentyltetrahydro-2H-pyran-2-one, (3E,5Z)-1,3,5-undecatriene, 10-undecenal, (9E)-9-undecenal (9Z)-9-undecenal, (Z)-4-decenal, (+-)-ethyl 2-methylpentanoate, 1,2-diallyldisulfane, 2-tridecenenitrile, 3-tridecenenitrile, , (+-)-2-ethyl-4,4-dimethyl-1,3-oxathiane, (+)-(3R,5Z)-3-methyl-5-cyclopentadecen-1-one, 3-(4-tert-butylphenyl)propanal, allyl (cyclohexyloxy)acetate, methylnaphthylketone, (+-)-(4E)-3-methyl-4-cyclopentadecen-1-one, (+-)-5E3-methyl-5-cyclopentadecen-1-one, cyclopropylmethyl 3-hexenoate, (4E)-4-methyl-5-(4-methylphenyl)-4-pentenal, (+-)-1-(5-propyl-1,3-benzodioxol-2-yl)ethanone, 4-methyl-2-pentylpyridine, (+-)-(E)-3-methyl-4-(2,6,6-trimethyl-2-cyclohexen-1-yl)-3-buten-2-one, (3aRS,5aSR,9aSR,9bRS)-3a,6,6,9a-tetramethyldodecahydronaphtho[2,1-b]furan, (2S,5R)-5-methyl-2-(2-propanyl)cyclohexanone oxime, 6-hexyltetrahydro-2H-pyran-2-one, (+-)-3-(3-isopropyl-1-phenyl)butanal, methyl 2-(3-oxo-2-pentylcyclopentyl)acetate, 1-(2,6,6-trimethyl-1-cyclohex-2-enyl)pent-1-en-3-one, indol, 7-propyl-2H,4H-1,5-benzodioxepin-3-one, ethyl praline, (4-methylphenoxy)acetaldehyde, ethyl tricyclo[5.2.1.0.^{2,6}]decane-2-carboxylate, (+)-(1'S,2S,E)-3,3-dimethyl-5-(2',2',3'-trimethyl-3'-cyclopenten-1'-yl)-4-penten-2-ol, (4E)-3,3-dimethyl-5-[(1R)-2,2,3-trimethyl-3-cyclopenten-1-yl]-4-penten-2-ol, 8-isopropyl-6-methyl-bicyclo[2.2.2]oct-5-ene-2-carbaldehyde, methylnonylacetaldehyde, 4-formyl-2-methoxyphenyl 2-methylpropanoate, (E)-4-decenal, (+-)-2-ethyl-4-(2,2,3-trimethyl-3-cyclopenten-1-yl)-2-buten-1-ol, (1R,5R)-4,7,7-trimethyl-6-thiabicyclo[3.2.1]oct-3-ene, (1R,4R,5R)-4,7,7-trimethyl-6-thiabicyclo[3.2.1]octane, (-)-(3R)-3,7-dimethyl-1,6-octadien-3-ol, (E)-3-phenyl-2-propenenitrile, 4-methoxybenzyl acetate, (E)-3-methyl-5-(2,2,3-trimethyl-3-cyclopenten-1-yl)-4-penten-2-ol, allyl (2/3-methylbutoxy)acetate, (+-)-(2E)-1-(2,6,6-trimethyl-2-cyclohexen-1-yl)-2-buten-1-one, (1E)-1-(2,6,6-trimethyl-1-cyclohexen-1-yl)-1-penten-3-one, and mixtures thereof.

According to an embodiment, perfume raw materials having a Log T<-4 are chosen in the group consisting of aldehydes, ketones, alcohols, phenols, esters lactones, ethers, epoxides, nitriles and mixtures thereof.

According to an embodiment, perfume raw materials having a Log T<-4 comprise at least one compound chosen in the group consisting of alcohols, phenols, esters lactones, ethers, epoxydes, nitriles and mixtures thereof, preferably in amount comprised between 20 and 70% by weight based on the total weight of the perfume raw materials having a Log T<-4.

According to an embodiment, perfume raw materials having a Log T<-4 comprise between 20 and 70% by weight of aldehydes, ketones, and mixtures thereof based on the total weight of the perfume raw materials having a Log T<-4.

The remaining perfume raw materials contained in the oil-based core may have therefore a Log T>-4.

According to an embodiment, the perfume raw materials having a Log T>-4 are chosen in the group consisting of ethyl 2-methylbutyrate, (E)-3-phenyl-2-propenyl acetate, (+-)-6/8-sec-butylquinoline, (+-)-3-(1,3-benzodioxol-5-yl)-2-methylpropanal, verdyl propionate, 1-(octahydro-2,3,8,8-tetramethyl-2-naphtalenyl)-1-ethanone, methyl 2-((1RS,2RS)-3-oxo-2-pentylcyclopentyl)acetate, (+-)-(E)-4-methyl-3-decen-5-ol, 2,4-dimethyl-3-cyclohexene-1-carbaldehyde, 1,3,3-trimethyl-2-oxabicyclo[2.2.2]octane, tetrahydro-4-methyl-2-(2-methyl-1-propenyl)-2H-pyran, dodecanal, 1-oxa-12/13-cyclohexadecen-2-one, (+-)-3-(4-isopropylphenyl)-2-methylpropanal, aldehyde C11, (+-)-2,6-dimethyl-7-octen-2-ol, allyl 3-cyclohexylpropanoate, (Z)-3-hexenyl acetate, 5-methyl-2-(2-propanyl)cyclohexanone, allyl heptanoate, 2-(2-methyl-2-propanyl)cyclohexyl acetate, 1,1-dimethyl-2-phenylethyl butyrate, geranyl acetate, neryl acetate, (+-)-1-phenylethyl acetate, 1,1-dimethyl-2-phenylethyl acetate, 3-methyl-2-butenyl acetate, ethyl 3-oxobutanoate, (2Z)-ethyl 3-hydroxy-2-butenoate, 8-p-menthanol, 8-p-menthanyl acetate, 1-p-menthanyl acetate, (+-)-2-(4-methyl-3-cyclohexen-1-yl)-2-propanyl acetate, (+-)-2-methylbutyl butanoate, 2-{(1S)-1-[(1R)-3,3-dimethylcyclohexyl]ethoxy}-2-oxoethyl propionate, 3,5,6-trimethyl-3-cyclohexene-1-carbaldehyde, 2,4,6-trimethyl-3-cyclohexene-1-carbaldehyde, 2-cyclohexylethyl acetate, octanal, ethyl butanoate, (+-)-(3E)-4-(2,6,6-trimethyl-1/2-cyclohexen-1-yl)-3-buten-2-one, 1-[(1RS,6SR)-2,2,6-trimethylcyclohexyl]-3-hexanol, 1,3,3-trimethyl-2-oxabicyclo[2.2.2]octane, 1,3,3-trimethyl-2-oxabicyclo[2.2.2]octane, ethyl hexanoate, undecanal, decanal, 2-phenylethyl acetate, (1S,2S,4S)-1,7,7-trimethylbicyclo[2.2.1]heptan-2-ol, (1S,2R,4S)-1,7,7-trimethylbicyclo[2.2.1]heptan-2-ol ), (+-)-3,7-dimethyl-3-octanol, 1-methyl-4-(2-propanylidene)cyclohexene, (+)-(R)-4-(2-methoxypropan-2-yl)-1-methylcyclohex-1-ene, verdyl acetate, (3R)-1-[(1R,68)-2,2,6-trimethylcyclohexyl]-3-hexanol, (3S)-1-[(1R,6S)-2,2,6-trimethylcyclohexyl]-3-hexanol, (3R)-1-[(1S,6S)-2,2,6-trimethylcyclohexyl]-3-hexanol, (+)-(1S,1'R)-2-[1-(3',3'-dimethyl-1'-cyclohexyl)ethoxy]-2-methylpropyl propanoate, and mixtures thereof.

According to an embodiment, the perfume formulation comprises
- 0 to 60 wt.% of a hydrophobic solvent (based on the total weight of the perfume formulation),
- 40 to 100 wt.% of a perfume oil (based on the total weight of the perfume formulation), wherein the perfume oil has at least two, preferably all of the following characteristics:
   ∘ at least 35%, preferably at least 40%, preferably at least 50%, more preferably at least 60% of perfuming ingredients having a log P above 3, preferably above 3.5,
   ∘ at least 20%, preferably at least 25%, preferably at least 30%, more preferably at least 40% of bulky materials of groups 1 to 6, preferably 3 to 6 as previously defined and
   ∘ at least 15%, preferably at least 20%, more preferably at least 25%, even more preferably at least 30% of high impact perfume materials having a Log T < -4,
optionally, further hydrophobic active ingredients.

According to a particular embodiment, the perfume comprises 0 to 60 wt.% of a hydrophobic solvent.

According to a particular embodiment, the hydrophobic solvent is a density balancing material preferably chosen in the group consisting of benzyl salicylate, benzyl benzoate, cyclohexyl salicylate, benzyl phenylacetate, phenylethyl phenylacetate, triacetin, ethyl citrate, methyl and ethyl salicylate, benzyl cinnamate, and mixtures thereof.

In a particular embodiment, the hydrophobic solvent has Hansen Solubility Parameters compatible with entrapped perfume oil.

The term "Hansen solubility parameter" is understood to a solubility parameter approach proposed by Charles Hansen used to predict polymer solubility and was developed around the basis that the total energy of vaporization of a liquid consists of several individual parts. To calculate the "weighted Hansen solubility parameter" one must combine the effects of (atomic) dispersion forces, (molecular) permanent dipole-permanent dipole forces, and (molecular) hydrogen bonding (electron exchange). The weighted Hansen solubility parameter" is calculated as (δD²+ δP²+ δH²)^{0.5}, wherein δD is the Hansen dispersion value (also referred to in the following as the atomic dispersion fore), δP is the Hansen polarizability value (also referred to in the following as the dipole moment), and δH is the Hansen Hydrogen-bonding ("h-bonding") value (also referred to in the following as hydrogen bonding). For a more detailed description of the parameters and values, see Charles Hansen, The Three Dimensional Solubility Parameter and Solvent Diffusion Coefficient, Danish Technical Press (Copenhagen, 1967).

Euclidean difference in solubility parameter between a fragrance and a solvent is calculated as (4*(δDₛₒₗᵥₑₙₜ-δD_{frgrance})² + (δPₛₒₗᵥₑₙₜ-δP_{frgrance})² + (δHₛₒₗᵥₑₙₜ-δH_{fragrance})²)^{0.5}, in which δDₛₒₗᵥₑₙₜ, δPₛₒₗᵥₑₙₜ, and δHₛₒₗᵥₑₙₜ, are the Hansen dispersion value, Hansen polarizability value, and Hansen h-bonding values of the solvent, respectively; and δD_{fragrance}, δP_{fragrance}, and δH_{fragrance} are the Hansen dispersion value, Hansen polarizability value, and Hansen h-bonding values of the fragrance, respectively.

In a particular embodiment, the perfume oil and the hydrophobic solvent have at least two Hansen solubility parameters selected from a first group consisting of: an atomic dispersion force (δD) from 12 to 20, a dipole moment (δP) from 1 to 8, and a hydrogen bonding (δH) from 2.5 to 11.

In a particular embodiment, the perfume oil and the hydrophobic solvent have at least two Hansen solubility parameters selected from a second group consisting of: an atomic dispersion force (δD) from 12 to 20, preferably from 14 to 20, a dipole moment (δP) from 1 to 8, preferably from 1 to 7, and a hydrogen bonding (δH) from 2.5 to 11, preferably from 4 to 11.

In a particular embodiment, at least 90% of the perfume oil, preferably at least 95% of the perfume oil, most preferably at least of 98% of the perfume oil has at least two Hansen solubility parameters selected from a first group consisting of: an atomic dispersion force (δD) from 12 to 20, a dipole moment (δP) from 1 to 8, and a hydrogen bonding (δH) from 2.5 to 11.

In a particular embodiment, the perfume oil and the hydrophobic solvent have at least two Hansen solubility parameters selected from a second group consisting of: an atomic dispersion force (δD) from 12 to 20, preferably from 14 to 20, a dipole moment (δP) from 1 to 8, preferably from 1 to 7, and a hydrogen bonding (δH) from 2.5 to 11, preferably from 4 to 11.

According to an embodiment, the perfuming formulation comprises a fragrance modulator (that can be used in addition to the hydrophobic solvent when present or as substitution of the hydrophobic solvent when there is no hydrophobic solvent).

Preferably, the fragrance modulator is defined as a fragrance material with
i. a vapor pressure of less than 0.0008 Torr at 22°C;
ii. a clogP of 3.5 and higher, preferably 4.0 and higher and more preferably 4.5
iii. at least two Hansen solubility parameters selected from a first group consisting of: an atomic dispersion force from 12 to 20, a dipole moment from 1 to 7, and a hydrogen bonding from 2.5 to 11,
iv. at least two Hansen solubility parameters selected from a second group consisting of: an atomic dispersion force from 14 to 20, a dipole moment from 1 to 8, and a hydrogen bonding from 4 to 11, when in solution with a compound having a vapor pressure range of 0.0008 to 0.08 Torr at 22°C.

Preferably, as examples the following ingredients can be listed as modulators but the list in not limited to the following materials: alcohol C12, oxacyclohexadec-12/13-en-2-one, 3-[(2',2',3'-trimethyl-3'-cyclopenten-1'-yl)methoxy]-2-butanol, cyclohexadecanone, (Z)-4-cyclopentadecen-1-one, cyclopentadecanone, (8Z)-oxacycloheptadec-8-en-2-one, 2-[5-(tetrahydro-5-methyl-5-vinyl-2-furyl)-tetrahydro-5-methyl-2-furyl]-2-propanol, muguet aldehyde, 1,5,8-trimethyl-13-oxabicyclo[10.1.0]trideca-4,8-diene, (+-)-4,6,6,7,8,8-hexamethyl-1,3,4,6,7,8-hexahydrocyclopenta[g]isochromene, (+)-(1S,2S,3S,5R)-2,6,6-trimethylspiro[bicyclo[3.1.1]heptane-3,1'-cyclohexane]-2'-en-4'-one, oxacyclohexadecan-2-one, 2-{(1S)-1-[(1R)-3,3-dimethylcyclohexyl]ethoxy}-2-oxoethyl propionate, (+)-(4R,4aS,6R)-4,4a-dimethyl-6-(1-propen-2-yl)-4,4a,5,6,7,8-hexahydro-2(3H)-naphthalenone, amylcinnamic aldehyde, hexylcinnamic aldehyde, hexyl salicylate, (1E)-1-(2,6,6-trimethyl-1-cyclohexen-1-yl)-1,6-heptadien-3-one, (9Z)-9-cycloheptadecen-1-one, and mixtures thereof.

According to a particular embodiment, the hydrophobic material is free of any active ingredient (such as perfume). According to this particular embodiment, it comprises, preferably consists of hydrophobic solvents, preferably chosen in the group consisting of isopropyl myristate, triglycerides (e.g. Neobee^{®} MCT oil, vegetable oils), D-limonene, silicone oil, mineral oil, and mixtures thereof with optionally hydrophilic solvents preferably chosen in the group consisting of 1,4-butanediol, benzyl alcohol, triethyl citrate, triacetin, benzyl acetate, ethyl acetate, propylene glycol (1,2-propanediol), 1,3-propanediol, dipropylene glycol, glycerol, glycol ethers and mixtures thereof .

The term "biocide" refers to a chemical substance capable of killing living organisms (e.g. microorganisms) or reducing or preventing their growth and/or accumulation. Biocides are commonly used in medicine, agriculture, forestry, and in industry where they prevent the fouling of, for example, water, agricultural products including seed, and oil pipelines. A biocide can be a pesticide, including a fungicide, herbicide, insecticide, algicide, molluscicide, miticide and rodenticide; and/or an antimicrobial such as a germicide, antibiotic, antibacterial, antiviral, antifungal, antiprotozoal and/or antiparasite.

As used herein, a "pest control agent" indicates a substance that serves to repel or attract pests, to decrease, inhibit or promote their growth, development or their activity. Pests refer to any living organism, whether animal, plant or fungus, which is invasive or troublesome to plants or animals, pests include insects notably arthropods, mites, spiders, fungi, weeds, bacteria and other microorganisms.

According to any one of the invention's embodiments, the hydrophobic material H1 represents between 10% and 60% w/w, or even between 15% and 45% w/w, by weight, relative to the total weight of the two-phases dispersion as obtained after step i).

### Continuous phase

According to the invention, the continuous phase in step a) comprises a polyol.

According to a particular embodiment, the content of water in the continuous phase is below or equal to 10% by weight based on the total weight of the continuous phase.

According to a particular embodiment, the continuous phase is free from water.

According to a particular embodiment, no water is added at any stage of the process.
Any polyol that is not soluble with the hydrophobic material H1 can be used in the present invention.
A single polyol or a mixture of polyols can be used.
The polyol can be chosen in the group consisting of glycerol,1,4-butanediol, ethylene glycol, 1,2-propylene glycol, 1,3-propylene glycol, triethanolamine, di(trimethylolpropane), ethylene glycol, glycerol, 1,4-butanediol, 1,2-hexanediol, 1,6-hexanediol, 2-ethyl-2-(hydroxymethyl)propane-1,3-diol (trimethylolpropane, TMP), 2,2-bis(hydroxymethyl)propane-1,3-diol (pentaerythritol), 2-amino-2-ethylpropane-1,3-diol, 2-amino-2-(hydroxymethyl)propane-1,3-diol, 2,2'-azanediylbis(ethan-1-ol), 2-aminopropane-1,3-diol, 2-amino-2-methylpropane-1,3-diol, polyphenols and mixtures thereof.

According to an embodiment, the polyol is added in an amount comprised between 10% and 90%, preferably between 25% and 60% by weight based on the total weight of the two-phases dispersion E1.

According to a particular embodiment, the polyol is a mixture of glycerol and propylene glycol, preferably 1,2 propylene glycol.

According to a particular embodiment, the polyol is a mixture of glycerol and 1,4-butanediol.

### Stabilizer S1

According to the invention, the oil phase O1 and/or the continuous phase comprise a stabilizer S1. The stabilizer S1 is preferably added in the continuous phase.

A single stabilizer S1 or mixture of stabilizers S1 can be used.

Said stabilizer can be an ionic or non-ionic emulsifier or a colloidal stabilizer. According to an embodiment, the stabilizer is a colloid stabilizer.

The stabilizer can be a molecular emulsifier (standard emulsion) or a colloidal particle stabilizer (Pickering emulsion). As colloidal particle stabilizer that can be used, one may cite for example calcium phosphate, silica, silicates, titanium dioxide, aluminium oxide, zinc oxide, iron oxide, mica, kaolin, montmorillonite, laponite, bentonite, perlite, dolomite, diatomite, vermiculite, hectorite, gibbsite, illite, kaolinite, aluminosilicates, gypsum, bauxite, magnesite, talc, magnesium carbonate, calcium carbonate, diatomaceous earth and mixtures thereof.

"Stabilizer" and "emulsifier" are used indifferently in the present invention.

Among the stabilizer S1 that can be used in the present invention, one may cite for example Tergitol 15-S-12 (secondary alcohol ethoxylate), Tween 80 (polyethylene glycol sorbitan monooleate), Tween 20 (polyethylene glycol sorbitan monooleate), Silica HKD H15 (hydrophobized silica particles); silk protein; Span^{®} 60 (sorbitan stearate), hydroxyapatite particles, cellulose derivatives such as hydroxypropylcellulose, and mixtures thereof.

According to an embodiment, the stabilizer S1 and/or S2 is chosen in the group consisting secondary alcohol ethoxylate, polyethylene glycol sorbitan monooleate, hydrophobized silica particles (hydrophobic silica particles); silk protein; sorbitan stearate, hydroxyapatite particles, polyglycerol polyricinoleate, sorbitane trioleate, polyglyceryl-3 diisostearate, cellulose derivatives such as hydroxypropylcellulose and mixtures thereof.

The stabilizer S1 is typically used in an amount comprised between 0.1 and 50%, preferably between 0.2 and 30%, most preferably between 0.5 to 20%, even more preferably between 1 to 10% by weight based on the total weight of the oil phase O1.

In another step of the process, the two-phases dispersion E1 is dispersed in an oil phase O2 comprising a hydrophobic material H2 and a stabilizer S2 to obtain a multiple dispersion E2;

The hydrophobic material H2 is defined as the hydrophobic material H1 defined previously and is preferably an inert solvent which is not soluble in the polyol, for example triglycerides (e.g. Neobee^{®} MCT oil, vegetable oils), isopropyl myristate, and mixtures thereof.

A single stabilizer S2 or mixture of stabilizers S2 can be used.

Among the stabilizer S2 that can be used in the present invention, one may cite for example PGPR90 (polyglycerol polyricinoleate), silica HKD H15 (hydrophobic silica particles), Span 85 (sorbitane trioleate), Makibase SEB (polyglyceryl-3 diisostearate), and mixtures thereof.

The stabilizer S2 is typically used in an amount comprised between 0.1 and 60%, preferably between 0.5 and 50%, most preferably 1 to 30%, even more preferably 1 to 10% by weight based on the total weight of the dispersion E1.

In another step of the process, at least a carboxylic acid derivative A3 is added into the multiple dispersion E2.

The carboxylic acid derivative A3 is chosen in the group consisting of acid, amide, ester, anhydride, acid chloride and mixtures thereof.

According to a particular embodiment, the carboxylic acid derivative A3 is an acyl chloride.

According to a particular embodiment, the acyl chloride has the following formula (I) 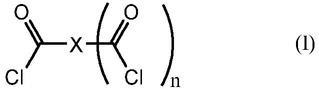
wherein n is an integer varying between 1 and 8, preferably between 1 and 6, more preferably between 1 and 4, and
wherein X is an (n+1)-valent C₂ to C₄₅ hydrocarbon group optionally comprising at least one group selected from (i) to (vi), 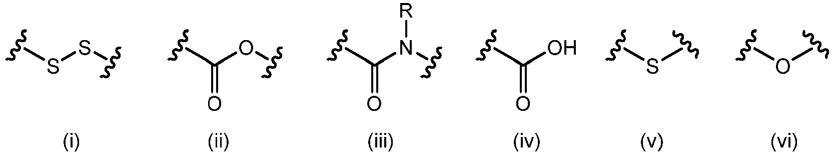 wherein R is a hydrogen atom or a methyl or ethyl group, preferably a hydrogen atom.

It is understood that by "... **hydrocarbon group** ..." it is meant that said group consists of hydrogen and carbon atoms and can be in the form of an aliphatic hydrocarbon, i.e. linear or branched saturated hydrocarbon (e.g. alkyl group), a linear or branched unsaturated hydrocarbon (e.g. alkenyl or alkynil group), a saturated cyclic hydrocarbon (e.g. cycloalkyl) or an unsaturated cyclic hydrocarbon (e.g. cycloalkenyl or cycloalkynyl), or can be in the form of an aromatic hydrocarbon, i.e. aryl group, or can also be in the form of a mixture of said type of groups, e.g. a specific group may comprise a linear alkyl, a branched alkenyl (e.g. having one or more carbon-carbon double bonds), a (poly)cycloalkyl and an aryl moiety, unless a specific limitation to only one type is mentioned. Similarly, in all the embodiments of the invention, when a group is mentioned as being in the form of more than one type of topology (e.g. linear, cyclic or branched) and/or being saturated or unsaturated (e.g. alkyl, aromatic or alkenyl), it is also meant a group which may comprise moieties having any one of said topologies or being saturated or unsaturated, as explained above. Similarly, in all the embodiments of the invention, when a group is mentioned as being in the form of one type of saturation or unsaturation, (e.g. alkyl), it is meant that said group can be in any type of topology (e.g. linear, cyclic or branched) or having several moieties with various topologies.

It is understood that with the term "... **a hydrocarbon group, optionally comprising** ..." it is meant that said hydrocarbon group optionally comprises heteroatoms to form ether, thioether, amine, nitrile or carboxylic acid groups. These groups can either substitute a hydrogen atom of the hydrocarbon group and thus be laterally attached to said hydrocarbon, or substitute a carbon atom (if chemically possible) of the hydrocarbon group and thus be inserted into the hydrocarbon chain or ring.

According to a particular embodiment, the acyl chloride is chosen from the group consisting of benzene-1,3,5-tricarbonyl trichloride (trimesoyl trichloride), benzene-1,2,4-tricarbonyl trichloride, benzene-1,2,4,5-tetracarbonyl tetrachloride, cyclohexane-1,3,5-tricarbonyl trichloride, isophthalyol dichloride, diglycolyl dichloride, terephthaloyl chloride, fumaryl dichloride, adipoyl dichloride, succinic acid dichloride (succinic dichloride or succinyl chloride or succinyl dichloride), propane-1,2,3-tricarbonyl trichloride, cyclohexane-1,2,4,5-tetracarbonyl tetrachloride, 2,2'-disulfanediyldisuccinyl dichloride, 2-(2-chloro-2-oxoethyl)sulfanylbutanedioyl dichloride, (4-chloro-4-oxobutanoyl)-L-glutamoyl dichloride, (S)-4-((1,5-dichloro-1,5-dioxopentan-2-yl)amino)-4-oxobutanoic acid, 2,2-bis[(4-chloro-4-oxo-butanoyl)oxymethyl]butyl 4-chloro-4-oxo-butanoate, [2-[2,2-bis[(4-chloro-4-oxo-butanoyl)oxymethyl]butoxymethyl]-2-[(4-chloro-4-oxo-butanoyl)oxymethyl]butyl] 4-chloro-4-oxo-butanoate, 2,2-bis[(2-chlorocarbonylbenzoyl)oxymethyl]butyl 2-chlorocarbonyl-benzoate, [2-[2,2-bis[(2-chlorocarbonylbenzoyl)oxymethyl]butoxymethyl]-2-[(2-chlorocarbonylbenzoyl)oxymethyl]butyl] 2-chlorocarbonylbenzoate, 4-(2,4,5-trichlorocarbonylbenzoyl)oxybutyl 2,4,5-trichlorocarbonyl-benzoate, propane-1,2,3-triyl tris(4-chloro-4-oxobutanoate), propane-1,2-diyl bis(4-chloro-4-oxobutanoate) and mixtures thereof.

According to a particular embodiment, the acyl chloride is chosen from the group consisting of benzene-1,2,4-tricarbonyl trichloride, benzene-1,2,4,5-tetracarbonyl tetrachloride, cyclohexane-1,3,5-tricarbonyl trichloride, isophthalyol dichloride, diglycolyl dichloride, terephthaloyl chloride, fumaryl dichloride, adipoyl dichloride, succinic dichloride, propane-1,2,3-tricarbonyl trichloride, cyclohexane-1,2,4,5-tetracarbonyl tetrachloride, 2,2'-disulfanediyldisuccinyl dichloride, 2-(2-chloro-2-oxo-ethyl)sulfanylbutanedioyl dichloride, (4-chloro-4-oxobutanoyl)-L-glutamoyl dichloride, (S)-4-((1,5-dichloro-1,5-dioxopentan-2-yl)amino)-4-oxobutanoic acid, 2,2-bis[(4-chloro-4-oxo-butanoyl)oxymethyl]butyl 4-chloro-4-oxo-butanoate, [2-[2,2-bis[(4-chloro-4-oxo-butanoyl)oxymethyl]butoxymethyl]-2-[(4-chloro-4-oxo-butanoyl)oxymethyl]butyl] 4-chloro-4-oxo-butanoate, 2,2-bis[(2-chlorocarbonylbenzoyl)oxymethyl]butyl 2-chlorocarbonyl-benzoate, [2-[2,2-bis[(2-chlorocarbonylbenzoyl)oxymethyl]butoxymethyl]-2-[(2-chlorocarbonylbenzoyl)oxymethyl]butyl] 2-chlorocarbonylbenzoate, 4-(2,4,5-trichlorocarbonylbenzoyl)oxybutyl 2,4,5-trichlorocarbonyl-benzoate, propane-1,2,3-triyl tris(4-chloro-4-oxobutanoate), propane-1,2-diyl bis(4-chloro-4-oxobutanoate), and mixtures thereof.

According to a particular embodiment, the acyl chloride is chosen from the group consisting of fumaryl dichloride, adipoyl dichloride, succinic dichloride (succinyl chloride), propane-1,2,3-triyl tris(4-chloro-4-oxobutanoate), propane-1,2-diyl bis(4-chloro-4-oxobutanoate), and mixtures thereof.

The carboxylic acid derivative A3 is preferably used in an amount from 0.1 to 50% by weight based on the total weight of the multiple dispersion E2.

According to an embodiment, the process comprises a step of adding at least a carboxylic acid derivative A1 into E1 after step i) and/or adding at least a carboxylic acid derivative A2 into the oil phase O1.

The definition and embodiments disclosed previously for carboxylic acid derivative A3 also apply for the carboxylic acid derivative A1 and for the carboxylic acid derivative A2.

According to a particular embodiment, the carboxylic acid derivative A1 and/or the carboxylic acid derivative A2 and/or the carboxylic acid derivative A3 is an acyl chloride.

According to a particular embodiment, the carboxylic acid derivative A1 and/or the carboxylic acid derivative A2 and/or the carboxylic acid derivative A3 can be the same or can be different.

According to a particular embodiment, the carboxylic acid derivative A3 is adipoyl chloride.

According to a particular embodiment, the carboxylic acid derivative A3 is succinyl chloride.

According to a particular embodiment, the carboxylic acid derivative A3 is adipoyl chloride and the carboxylic acid derivative A1 is trimesoyl trichloride.

According to a particular embodiment, the carboxylic acid derivative A3 is adipoyl chloride and the carboxylic acid derivative A1 is succinyl chloride
According to a particular embodiment:
- the polyol is a mixture of glycerol and 1,4-butanediol.
- the carboxylic acid derivative A3 is succinyl chloride or adipoyl chloride, and
- the stabilizer S1 is hydrophobized silica particles.

In another step of the process, conditions sufficient are applied to induce interfacial polymerization and form polyester microcapsules in the form of a slurry.

This step allows ending up with microcapsules in the form of a slurry. According to a preferred embodiment, to enhance the kinetics, said step is performed at a temperature comprised between 5 and 90°C, possibly under pressure, for 1 to 30 hours. More preferably it is performed at between 10 and 80°C for between 30 minutes and 5 hours. However, this step (called curing step) can be carried out at room temperature.

According to a particular embodiment of the invention, at the end of step iv) or during step iv) one may also add to the invention's slurry a polymer selected from the group consisting of a non-ionic polysaccharide, a cationic polymer, a polysuccinimide derivative (as described for instance in WO2021185724) and mixtures thereof to form an outer coating to the microcapsule.

Non-ionic polysaccharide polymers are well known to a person skilled in the art and are described for instance in WO2012/007438 page 29, lines 1 to 25 and in WO2013/026657 page 2, lines 12 to 19 and page 4, lines 3 to 12. Preferred non-ionic polysaccharides are selected from the group consisting of locust bean gum, xyloglucan, guar gum, hydroxypropyl guar, hydroxypropyl cellulose and hydroxypropyl methyl cellulose.

Cationic polymers are well known to a person skilled in the art. Preferred cationic polymers have cationic charge densities of at least 0.5 meq/g, more preferably at least about 1.5 meq/g, but also preferably less than about 7 meq/g, more preferably less than about 6.2 meq/g. The cationic charge density of the cationic polymers may be determined by the Kjeldahl method as described in the US Pharmacopoeia under chemical tests for Nitrogen determination. The preferred cationic polymers are chosen from those that contain units comprising primary, secondary, tertiary and/or quaternary amine groups that can either form part of the main polymer chain or can be borne by a side substituent directly connected thereto. The weight average (Mw) molecular weight of the cationic polymer is preferably between 10,000 and 3.5M Dalton, more preferably between 50,000 and 1.5M Dalton. According to a particular embodiment, one will use cationic polymers based on acrylamide, methacrylamide, N-vinylpyrrolidone, quaternized N,N-dimethylaminomethacrylate, diallyldimethylammonium chloride, quaternized vinylimidazole (3-methyl-1-vinyl-1H-imidazol-3-ium chloride), vinylpyrrolidone, acrylamidopropyltrimonium chloride, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. Preferably copolymers shall be selected from the group consisting of polyquaternium-5, polyquaternium-6, polyquaternium-7, polyquaternium10, polyquaternium-11, polyquaternium-16, polyquaternium-22, polyquaternium-28, polyquaternium-43, polyquaternium-44, polyquaternium-46, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. As specific examples of commercially available products, one may cite Salcare^{®} SC60 (cationic copolymer of acrylamidopropyltrimonium chloride and acrylamide, origin: BASF) or Luviquat^{®}, such as the PQ 11N, FC 550 or Style (polyquaternium-11 to 68 or quaternized copolymers of vinylpyrrolidone origin: BASF), or also the Jaguar^{®} (C13S or C17, origin Rhodia).

According to any one of the above embodiments of the invention, there is added an amount of polymer described above comprised between about 0% and 5% w/w, or even between about 0.1% and 2% w/w, percentage being expressed on a w/w basis relative to the total weight of the slurry as obtained after step iv). It is clearly understood by a person skilled in the art that only part of said added polymers will be incorporated into/deposited on the microcapsule shell.

According to an embodiment, no amine, preferably no tertiary amine is added at any step of the process.

According to a particular embodiment, the process comprises an additional step consisting of adding a hydrophobic material H3 in the slurry. The hydrophobic material H3 is preferably added in an amount comprised between 20% and 80%, preferably between 30% and 75% by weight based on the total weight of the slurry as obtained after step iv).

The hydrophobic material H3 is defined as the hydrophobic material H1 defined previously and is preferably a perfume.

According to a particular embodiment, the process comprises an additional step consisting of removing the hydrogen chloride (HCl) from the slurry. Indeed, HCl might be generated during the process and can be removed for example by using a gas flow or by using a base.

Also disclosed is a process for preparing a microcapsule powder comprising the steps as defined above and an additional step consisting of submitting the slurry obtained in step iv) or v) to a drying, like spray-drying, to provide the microcapsules as such, i.e. in a powdery form. It is understood that any standard method known by a person skilled in the art to perform such drying is also applicable. In particular the slurry may be spray-dried preferably in the presence of a polymeric carrier material such as polyvinyl acetate, polyvinyl alcohol, dextrins, natural or modified starch, vegetable gums, pectins, xanthans, alginates, carragenans or cellulose derivatives to provide microcapsules in a powder form.

However, one may cite also other drying method such as the extrusion, plating, spray granulation, the fluidized bed, or even a drying at room temperature using materials (carrier, desiccant) that meet specific criteria as disclosed in WO2017/134179.

According to a particular embodiment, the carrier material contains free perfume oil which can be the same or different from the perfume from the core of the microcapsules.

### Multiple capsule system

According to an embodiment, the microcapsules of the invention (first type of microcapsule) can be used in combination with a second type of microcapsules.

Also disclosed is a microcapsule delivery system comprising:
- the microcapsules of the present invention as a first type of microcapsules, and
- a second type of microcapsules, wherein the first type of microcapsules and the second type of microcapsules differ in their hydrophobic material and/or their carrier material and/or in their coating material.

### Polyester microcapsule

The embodiments disclosed previously for defining components in the process also apply for the microcapsule.

A second object of the invention is a polyester microcapsule slurry obtainable by the process as defined above.

Also disclosed is a polyester microcapsule comprising:
- a core comprising an oil phase, wherein the oil phase comprises a hydrophobic material H1, and
- a carrier comprising the reaction product between at least one carboxylic acid derivative A3 and a polyol,
wherein the core is entrapped in the carrier.

According to the invention, the core comprises a two-phases dispersion made of the polyol and the hydrophobic material H1.
The microcapsule of the invention can be a core-shell type or a matrix type, depending notably on the nature of the carboxylic acid derivative A3.
According to an embodiment, the microcapsule is in the form of a matrix.

In this embodiment, the carrier material is made of polyester like poly(glycerol-co-succinate), poly(propylene glycol-co-succinate), polyester like poly(glycerol-co-propylene glycol-co-succinate), poly(glycerol-co-adipate), poly(propylene glycol-co-adipate), polyester like poly(glycerol-co-propylene glycol-co-adipate), and mixtures thereof.

According to an embodiment, the microcapsule is in the form of core-shell microcapsule.

In this embodiment, the carrier material is made of polyester like poly(glycerol-co-succinate), poly(propylene glycol-co-succinate), polyester like poly(glycerol-co-propylene glycol-co-succinate), poly(glycerol-co-adipate), poly(propylene glycol-co-adipate), polyester like poly(glycerol-co-propylene glycol-co-adipate), and mixtures thereof.

According to a particular embodiment, an additional polyester shell made from the reaction between a carboxylic acid derivative A1 and/or carboxylic acid derivative A2 with the polyol is formed at the interface between the core and the carrier material made of polyester.

According to a particular embodiment, stabilizers S1 and S2 are at the interface between the core and the carrier material made of polyester.

Also disclosed is a polyester microcapsule slurry comprising at least one polyester microcapsule made of:
- a core comprising an oil phase, wherein the oil phase comprises a hydrophobic material H1, and
- a carrier comprising the reaction product between an carboxylic acid derivative A3 and a polyol, wherein the core is entrapped in the carrier.

Embodiments disclosed for the microcapsules previously also apply for the microcapsule slurry.

According to this embodiment, the microcapsules are dispersed in a hydrophobic material H2, preferably an inert solvent which is not soluble in the polyol, for example triglycerides (e.g. Neobee^{®} MCT oil, vegetable oils), isopropyl myristate, and mixtures thereof.

According to an embodiment, the microcapsule slurry comprises a hydrophobic material H3, preferably a perfume freely dispersed in the slurry.

In a particular embodiment, the shell material comprises a biodegradable material.

In a particular embodiment, the shell has a biodegradability of at least 40%, preferably at least 45%, 50%, 60%, 65%, 70%, 75%, 80%, 85%, 90%, 95% or 98%, within 60 days according to OECD301F.

In a particular embodiment, the core-shell microcapsule has a biodegradability of at least 40 %, preferably at least 60 %, preferably at least 65%, 70%, 75%, 80%, 85%, 90%, 95% or 98% within 60 days according to OECD301F.

Thereby it is understood that the core-shell microcapsule including all components, such as the core, shell and optionally coating may have a biodegradability of at least 40 %, preferably at least 60 %, preferably at least 65%, 70%, 75%, 80%, 85%, 90%, 95% or 98% within 60 days according to OECD301F.

OECD301F is a standard test method on the biodegradability from the Organization of Economic Co-operation and Development.

A typical method for extracting the shell for measuring the biodegradability is disclosed in Gasparini and all in Molecules 2020, 25,718*.*

### Perfuming composition/consumer products

The microcapsules of the invention can be used in combination with active ingredients. Also disclosed is a composition comprising:
(i) microcapsules or microcapsule slurry as defined above;
(ii) an active ingredient, preferably chosen in the group consisting of a cosmetic ingredient, skin caring ingredient, perfume ingredient, flavor ingredient, malodour counteracting ingredient, bactericide ingredient, fungicide ingredient, pharmaceutical or agrochemical ingredient, a sanitizing ingredient, an insect repellent or attractant, and mixtures thereof.

The microcapsules of the invention can be used for the preparation of perfuming or flavouring compositions.

The capsules of the invention show very good performance in terms of stability in challenging medium.

Also disclosed is a perfuming composition comprising:
(i) microcapsules or microcapsule slurry as defined above, wherein the oil comprises a perfume;
(ii) at least one ingredient selected from the group consisting of a perfumery carrier, a perfumery co-ingredient and mixtures thereof;
(iii) optionally at least one perfumery adjuvant.

As liquid perfumery carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the most commonly used. For the compositions which comprise both a perfumery carrier and a perfumery co-ingredient, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company). By "perfumery co-ingredient" it is meant here a compound, which is used in a perfuming preparation or a composition to impart a hedonic effect and which is not a microcapsule as defined above. In other words such a co-ingredient, to be considered as being a perfuming one, must be recognized by a person skilled in the art as being able to at least impart or modify in a positive or pleasant way the odor of a composition, and not just as having an odor.

The nature and type of the perfuming co-ingredients present in the perfuming composition do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds also known as properfume or profragrance. Non-limiting examples of suitable properfumes may include
4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, trans-3-(dodecylthio)-1-(2,6,6-trimethyl-3-cyclohexen-1-yl)-1-butanone, 2-(dodecylthio)octan-4-one, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1-(((Z)-hex-3-en-1-yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, 2-(1-phenethoxyprop-1-en-2-yl)naphthalene, (2-phenethoxyvinyl)benzene, 2-(1-((3,7-dimethyloct-6-en-1-yl)oxy)prop-1-en-2-yl)naphthalene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 3-methoxy-4-((2-methoxy-2-phenylvinyl)oxy)benzaldehyde, 4-((2-(hexyloxy)-2-phenylvinyl)oxy)-3-methoxybenzaldehyde or a mixture thereof.

By **"perfumery adjuvant"** we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

Preferably, the perfuming composition according to the invention comprises between 0.01 and 30 % by weight of microcapsules as defined above.

The invention's microcapsules can advantageously be used in many application fields and used in consumer products. Microcapsules can be used in liquid form applicable to liquid consumer products as well as in powder form, applicable to powder consumer products.

According to a particular embodiment, the consumer product as defined above is liquid and comprises:
a) from 2 to 65% by weight, relative to the total weight of the consumer product, of at least one surfactant;
b) water or a water-miscible hydrophilic organic solvent; and
c) microcapsule slurry or microcapsules as defined above,
d) optionally non-encapsulated perfume.

According to a particular embodiment, the consumer product as defined above is in a powder form and comprises:
a) from 2 to 65% by weight, relative to the total weight of the consumer product, of at least one surfactant;
b) microcapsule powder as defined above.
c) optionally perfume powder that is different from the microcapsules defined above.

In the case of microcapsules including a perfume oil-based core, the products of the invention, can in particular be of used in perfumed consumer products such as product belonging to fine fragrance or "functional" perfumery. Functional perfumery includes in particular personal-care products including hair-care, body cleansing, skin care, hygiene-care as well as home-care products including laundry care and air care. Consequently, another object of the present invention consists of a perfumed consumer product comprising as a perfuming ingredient, the microcapsules defined above or a perfuming composition as defined above. The perfume element of said consumer product can be a combination of perfume microcapsules as defined above and free or non-encapsulated perfume, as well as other types of perfume microcapsule than those here-disclosed.

In particular a liquid consumer product comprising:
a) from 2 to 65% by weight, relative to the total weight of the consumer product, of at least one surfactant;
b) water or a water-miscible hydrophilic organic solvent; and
c) a perfuming composition as defined above is another object of the invention.

Also a powder consumer product comprising
(a) from 2 to 65% by weight, relative to the total weight of the consumer product, of at least one surfactant; and
(b) a perfuming composition as defined above is part of the invention.

The invention's microcapsules can therefore be added as such or as part of a perfuming composition in a perfumed consumer product.

For the sake of clarity, it has to be mentioned that, by "perfumed consumer product" it is meant a consumer product which is expected to deliver among different benefits a perfuming effect to the surface to which it is applied (e.g. skin, hair, textile, paper, or home surface) or in the air (air-freshener, deodorizer etc). In other words, a perfumed consumer product according to the invention is a manufactured product which comprises a functional formulation also referred to as "base", together with benefit agents, among which an effective amount of microcapsules according to the invention.

The nature and type of the other constituents of the perfumed consumer product do not warrant a more detailed description here, which in any case would not be exhaustive, the skilled person being able to select them on the basis of his general knowledge and according to the nature and the desired effect of said product. Base formulations of consumer products in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Non-limiting examples of suitable perfumed consumer product can be a perfume, such as a fine perfume, a cologne, an after-shave lotion, a body-splash; a fabric care product, such as a liquid or solid detergent, tablets and unit dose (single chamber or multi chambers), a fabric conditioner, a dryer sheet, a fabric refresher, an ironing water, or a bleach; a personal-care product, such as a hair-care product (e.g. a shampoo, hair conditioner, a colouring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product); an air care product, such as an air freshener or a "ready to use" powdered air freshener; or a home care product, such all-purpose cleaners, liquid or power or tablet dishwashing products, toilet cleaners or products for cleaning various surfaces, for example sprays & wipes intended for the treatment / refreshment of textiles or hard surfaces (floors, tiles, stone-floors etc.); a hygiene product such as sanitary napkins, diapers, toilet paper.

Fabric softener or fabric conditioner are used indifferently in the present invention.

Another object of the invention is a consumer product comprising:
- a personal care active base, and
- microcapsules as defined above,
wherein the consumer product is in the form of a personal care composition.

Personal care active base in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.
The personal care composition is preferably chosen in the group consisting of a hair-care product (e.g. a shampoo, hair conditioner, a colouring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product);

Another object of the invention is a consumer product comprising:
a home care or a fabric care active base, and
microcapsules as defined above,
wherein the consumer product is in the form of a home care or a fabric care composition.

Home care or fabric care active base in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Preferably, the consumer product comprises from 0.1 to 15 wt%, more preferably between 0.2 and 5 wt% of the microcapsules of the present invention, these percentages being defined by weight relative to the total weight of the consumer product. Of course the above concentrations may be adapted according to the benefit effect desired in each product.

For liquid consumer product mentioned below, by "active base", it should be understood that the active base includes active materials (typically including surfactants) and water.

For solid consumer product mention below , by "active base", it should be understood that the active base includes active materials (typically including surfactants) and auxiliary agents (such as bleaching agents, buffering agent; builders; soil release or soil suspension polymers; granulated enzyme particles, corrosion inhibitors, antifoaming, sud suppressing agents; dyes, fillers, and mixtures thereof).

### Fabric softener

Also disclosed is a consumer product in the form of a fabric softener composition comprising:
- a fabric softener active base; preferably comprising at least one active material chosen in the group consisting of dialkyl quaternary ammonium salts, dialkyl ester quaternary ammonium salts (esterquats), Hamburg esterquat (HEQ), TEAQ (triethanolamine quat), silicones and mixtures thereof, the active base being used preferably in an amount comprised between 85 and 99.95% by weight based on the total weight of the composition,
- a microcapsule slurry as defined above, preferably in an amount comprised between 0.05 to 15 wt%, more preferably between 0.1 and 5 wt% by weight based on the total weight of the composition,
- optionally free perfume oil.

### Liquid detergent

Also disclosed is a consumer product in the form of a liquid detergent composition comprising:
- a liquid detergent active base; preferably comprising at least one active material chosen in the group consisting of anionic surfactant such as alkylbenzenesulfonate (ABS), secondary alkyl sulfonate (SAS), primary alcohol sulfate (PAS), lauryl ether sulfate (LES), methyl ester sulfonate (MES) and nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides, the active base being used preferably in an amount comprised between 85 and 99.95% by weight based on the total weight of the composition,
- a microcapsule slurry as defined above, preferably in an amount comprised between 0.05 to 15 wt%, more preferably between 0.1 and 5 wt% by weight based on the total weight of the composition,
- optionally free perfume oil.

### Solid detergent

Also disclosed is a consumer product in the form of a solid detergent composition comprising:
- a solid detergent active base; preferably comprising at least one active material chosen in the group consisting of anionic surfactant such as alkylbenzenesulfonate (ABS), secondary alkyl sulfonate (SAS), primary alcohol sulfate (PAS), lauryl ether sulfate (LES), methyl ester sulfonate (MES) and nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides, the active base being used preferably in an amount comprised between 85 and 99.95% by weight based on the total weight of the composition,
- a microcapsule powder or microcapsule slurry as defined above, preferably in an amount comprised between 0.05 to 15 wt%, more preferably between 0.1 and 5 wt% by weight based on the total weight of the composition,
- optionally free perfume oil.

### Shampoo/shower gel

Also disclosed is a consumer product in the form of a shampoo or a shower gel composition comprising:
- a shampoo or a shower gel active base; preferably comprising at least one active material chosen in the group consisting of sodium alkylether sulfate, ammonium alkylether sulfates, alkylamphoacetate, cocamidopropyl betaine, cocamide MEA, alkylglucosides and aminoacid based surfactants and mixtures thereof, the active base being used preferably in an amount comprised between 85 and 99.95% by weight based on the total weight of the composition,
- a microcapsule slurry as defined above, preferably in an amount comprised between 0.05 to 15 wt%, more preferably between 0.1 and 5 wt% by weight based on the total weight of the composition,
- optionally free perfume oil.

### Rinse-Off Conditioner

Also disclosed is a consumer product in the form of a rinse-off conditioner composition comprising:
- a rinse-off conditioner active base; preferably comprising at least one active material chosen in the group consisting of cetyltrimonium chloride, stearyl trimonium chloride, benzalkonium chloride, behentrimonium chloride and mixture thereof, the active base being used preferably in an amount comprised between 85 and 99.95% by weight based on the total weight of the composition,
- a microcapsule slurry as defined above, preferably in an amount comprised between 0.05 to 15 wt%, more preferably between 0.1 and 5 wt% by weight based on the total weight of the composition,
- optionally free perfume oil.

### Solid scent booster

Also disclosed is a consumer product in the form of a solid scent booster composition comprising:
- a solid carrier, preferably chosen in the group consisting of urea, sodium chloride, sodium sulphate, sodium acetate, zeolite, sodium carbonate, sodium bicarbonate, clay, talc, calcium carbonate, magnesium sulfate, gypsum, calcium sulfate, magnesium oxide, zinc oxide, titanium dioxide, calcium chloride, potassium chloride, magnesium chloride, zinc chloride, saccharides such as sucrose, mono-, di-, and polysaccharides and derivatives such as starch, cellulose, methyl cellulose, ethyl cellulose, propyl cellulose, polyols/sugar alcohols such as sorbitol, maltitol, xylitol, erythritol, and isomalt, PEG, PVP, citric acid or any water soluble solid acid, fatty alcohols or fatty acids and mixtures thereof,
- a microcapsule slurry as defined above, in a powdered form, preferably in an amount comprised between 0.05 to 15 wt%, more preferably between 0.1 and 5 wt% by weight based on the total weight of the composition,
- optionally free perfume oil.

### Liquid scent booster

Also disclosed is a consumer product in the form of a liquid scent booster composition comprising:
- an aqueous phase,
- a surfactant system essentially consisting of one or more than one non-ionic surfactant, wherein the surfactant system has a mean HLB between 10 and 14, preferably chosen in the group consisting of ethoxylated aliphatic alcohols, POE/PPG (polyoxyethylene and polyoxypropylene) ethers, mono and polyglyceryl esters, sucrose ester compounds, polyoxyethylene hydroxylesters, alkyl polyglucosides, amine oxides and combinations thereof;
- a linker chosen in the group consisting of alcohols, salts and esters of carboxylic acids, salts and esters of hydroxyl carboxylic acids, fatty acids, fatty acid salts, glycerol fatty acids, surfactant having an HLB less than 10 and mixtures thereof, and

- a microcapsule slurry as defined above, in the form of a slurry, preferably in an amount comprised between 0.05 to 15 wt%, more preferably between 0.1 and 5 wt% by weight based on the total weight of the composition,
- optionally free perfume oil.

### Hair coloration

Also disclosed is a consumer product in the form of an oxidative hair coloring composition comprising:
- an oxidizing phase comprising an oxidizing agent and an alkaline phase comprising an alkakine agent, a dye precursor and a coupling compound; wherein said dye precursor and said coupling compound form an oxidative hair dye in the presence of the oxidizing agent, preferably in an amount comprised between 85 and 99.95% by weight based on the total weight of the composition,
- microcapsules slurry as defined above, preferably in an amount comprised between 0.05 to 15 wt%, more preferably between 0.1 and 5 wt% by weight based on the total weight of the composition,
- optionally free perfume oil

### Perfuming composition

According to a particular embodiment, the consumer product is in the form of a perfuming composition comprising:
- 0.1 to 30%, preferably 0.1 to 20% of microcapsules, preferably in the form of a slurry, as defined previously,
- 0 to 40%, preferably 3-40% of perfume, and
- 20-90%, preferably 40-90% of ethanol, by weight based on the total weight of the perfuming composition.

The invention will now be further described by way of examples. It will be appreciated that the invention as claimed is not intended to be limited in any way by these examples.

### Examples

**Table 1: Materials used**

| **Ingredients** | **Effective Amount of Ingredient (% wt)^{a)}** |
|---|---|
| Perfume oil ¹⁾ | 20 |
| Trimesoyl trichloride ²⁾ | 0.4 |
| Tween 80 ³⁾ | 0.4 |
| Glycerol ⁴⁾ | 20 |
| Silica HKD H15 ⁵⁾ | 2 |
| Neobee ⁶⁾ | 46 |
| Adipoyl chloride ⁷⁾ | 10 |
| Fumaryl dichloride ⁸⁾ | 10 |
| Succinyl dichloride ⁹⁾ | 10 |
| Tween 20 ³⁾ | 0.4 |
| PGPR90 ¹⁰⁾ | 1 |
| Makibase SEB ¹¹⁾ | 8 |

| | |
|---|---|
| 1) Perfume oil A; origin: Firmenich SA, Switzerland 2) 1,3,5-Benzene tricarbonyle chloride, origin: Sigma Aldrich, Switzerland 3) Polyethylene glycol sorbitan monooleate, origin: Sigma Aldrich, Switzerland 4) 1,2,3-Propanetriol, origin: Sigma Aldrich, Switzerland 5) Hydrophobic silica particles - Silica HKD H15 nanoparticles possessing 50% silanol groups, origin: Wacker Silicones, Germany 6) Neobee M5, origin: Firmenich SA, Switzerland 7) Adipyl chloride, origin: Sigma Aldrich, Switzerland 8) Fumaryl chloride, origin: Sigma Aldrich, Switzerland 9) Succinyl chloride, origin: Sigma Aldrich, Switzerland 10)Polyglycerol Polyricinoleate, origin: Danisco, Danemark 11)Polyglyceryl-3 diisostearate, origin: Daito Kasei, Japan | |

**Table 2: Perfume oil A composition**

| **Raw Materials** | **% in oil** |
|---|---|
| 2,4-Dimethyl-3-cyclohexene-1-carbaldehyde | 3.30% |
| Allyl Heptanoate | 5.50% |
| Allyl amyl glycolate | 10.99% |
| Delta Damascone | 1.65% |
| Verdyl acetate | 20.30% |
| Methyl dihydrojasmonate ¹⁾ | 4.95% |
| 1-(octahydro-2,3,8,8-tetramethyl-2-naphtalenyl)-1-ethanone ²⁾ | 16.49% |
| Ald. Hexylcinnamique | 9.89% |
| Ethyl-2-methylvalerate | 3.3% |
| (+-)-2-methyl-3-[4-(2-methyl-2-propanyl)phenyl]propanal | 21.98% |
| (Z)-hex-3-en-1-yl butyrate | 1.1% |
| (-)-(8R)-8,12-epoxy-13,14,15,16-tetranorlabdane³⁾ | 0.55% |
| Total | 100% |

| | |
|---|---|
| 1) Firmenich SA, Geneva, Switzerland 2) International Flavors & Fragrances, USA 3) Firmenich SA, Geneva, Switzerland | |

### Example 1

### Preparation of microcapsules according to the invention (addition of carboxylic acid derivative A3 in the multiple emulsion E2)

**General protocol:** Perfume oil A was dispersed in a solution of Tween 80 (S1) in glycerol with the help of an ultra turrax at 12,000 rpm for 5 min. The resulting emulsion was dispersed in a dispersion of silica HKD H15 (S2) in Neobee at 12,000 rpm with the help of a an ultra turrax for 5 min at room temperature to afford a multiple emulsion. Adipoyl chloride was added dropwise to the multiple emulsion and the reaction mixture was stirred at 30°C for 45 h.

**Table 3: Microcapsule composition**

| Capsule | Perfume A (g) | Glycerol (g) | Tween 80 (g) | Neobee M5 (g) | Silica HKD H15 (g) | Adipoyl chloride (g) |
|---|---|---|---|---|---|---|
| 1A | 5 | 9.8 | 0.2 | 33.95 | 1.05 | 1 |
| 1B | 9.7 | 19.01 | 0.39 | 29.1 | 0.9 | 1 |
| 1C | 5 | 9.8 | 0.2 | 33.95 | 1.05 | 1.02 |
| 1D | 5 | 9.8 | 0.2 | 33.95 | 1.05 | 7.44 |
| 1E | 5 | 9.8 | 0.2 | 33.95 | 1.05 | 10.03 |
| 1F | 5 | 9.8 | 0.2 | 33.95 | 1.05 | 2.63 |
| 1G | 5 | 9.8 | 0.2 | 33.95 | 1.05 | 5.02 |

| | | | | | | |
|---|---|---|---|---|---|---|
| Capsules of examples 1H to 1M were prepared with Tween 20 (S1) and Makibase SEB (S2) | | | | | | |

**Table 4: Microcapsule composition**

| Capsule | Perfume A (g) | Glycerol (g) | Tween 20 (g) | Neobee M5 (g) | Makibase SEB (g) | Adipoyl chloride (g) |
|---|---|---|---|---|---|---|
| 1H | 0.25 | 0.98 | 0.02 | 4.5 | 0.5 | 2.9 |
| 1I | 0.25 | 0.98 | 0.02 | 4.5 | 0.5 | 0.73 |
| 1J | 0.25 | 0.98 | 0.02 | 4.5 | 0.15 | 1.02 |
| 1K | 1.28 | 5.00 | 0.10 | 20.25 | 2.25 | 0.77 |
| 1L | 3.15 | 12.34 | 0.25 | 56.68 | 6.30 | 1.89 |

| | | | | | | |
|---|---|---|---|---|---|---|
| Capsules of example 1M were prepared with Tween 20 (S1) and Polyglycerol Polyricinoleate (PGPR90) (S2) | | | | | | |

**Table 5: Microcapsule composition**

| Capsule | Perfume A (g) | Glycerol (g) | Tween 20 (g) | Neobee M5 (g) | PGPR90 (g) | Adipoyl chloride (g) |
|---|---|---|---|---|---|---|
| 1M | 2.5 | 9.80 | 0.20 | 27.00 | 3.00 | 0.13 |

### Example 2

### Preparation of microcapsules according to the invention (addition of carboxylic acid derivative A3 in the multiple emulsion E2 and carboxylic acid derivative A2 in the oil phase O1)

**General protocol:** Perfume oil A and trimesoyl trichloride was dispersed in a solution of Tween 80 (S1) in glycerol with the help of an ultra turrax at 12,000 rpm for 5 min. The resulting emulsion was dispersed in a dispersion of silica HKD H15 (S2) in Neobee at 12,000 rpm with the help of a an ultra turrax for 5 min at room temperature to afford a multiple emulsion. Adipoyl chloride was added dropwise to the multiple emulsion and the reaction mixture was stirred at 30°C for 45 h.

**Table 6: Microcapsule composition**

| Capsule | Perfume A (g) | Trimesoyl chloride (g) | Glycerol (g) | Tween 80 (g) | Neobee M5 (g) | Silica HKD H15 (g) | Adipoyl chloride (g) |
|---|---|---|---|---|---|---|---|
| 2A | 5 | 0.10 | 9.80 | 0.20 | 33.95 | 1.05 | 1.02 |
| 2B | 5 | 0.10 | 9.80 | 0.20 | 33.95 | 1.05 | 5.00 |
| 2C | 5 | 0.35 | 9.8 | 0.2 | 33.95 | 1.05 | 10.00 |
| 2D | 5 | 0.35 | 9.8 | 0.2 | 33.95 | 1.05 | 15.00 |
| 2E | 5 | 0.35 | 9.8 | 0.2 | 33.95 | 1.05 | 20.00 |

### Example 3

### Preparation of microcapsules according to the invention (addition of carboxylic acid derivative A3 in the multiple emulsion E2 and carboxylic acid derivative A1 in the emulsion E1)

**General protocol:** Tween 20 (S1) was dissolved in a mixture of glycerol and 1,2-propylene glycol. Perfume oil A was dispersed in this solution with the help of an ultra turrax at 24,000 rpm for 2 min at 0°C. Succinyl chloride was added to the first emulsion at room temperature. The reaction mixture was stirred at room temperature for different periods of time. The resulting emulsion was dispersed in a solution of PGPR90 (S2) in Neobee at 550 rpm with the help of a mechanical stirrer for 10 min at room temperature to afford a multiple emulsion. Adipoyl chloride was added dropwise to the multiple emulsion over the course of 2 h, and the reaction mixture was stirred at 25°C for 24 h.

**Table 7: Microcapsule composition**

| Capsule | Perfume A (g) | 1,2-propylene glycol (g) | Glycerol (g) | Tween 20 (g) | Succinyl chloride (g) | t (h) | Neobee M5 (g) | PGPR 90 (g) | Adipoyl chloride (g) |
|---|---|---|---|---|---|---|---|---|---|
| 3A | 5 | 4.00 | 6.80 | 0.20 | 6.44 | 2.5 | 54.00 | 6.00 | 2.40 |
| 3B | 5 | 4.00 | 6.80 | 0.20 | 6.44 | 1.0 | 54.00 | 6.00 | 2.40 |
| 3C | 0.83 | 6.80 | 0.50 | 0.03 | 0.69 | 0.1 | 9.00 | 1.00 | 0.44 |
| 3C | 5.00 | 6.80 | 3.00 | 0.20 | 12.80 | 5.5 | 49.00 | 1.00 | 1.00 |
| 3D | 5.00 | 3.00 | 6.80 | 0.20 | 1.50 | 1.0 | 20.00 | 0.40 | 1.15 |

**Capsules 3E:** Tween 20 (S1) (0.20 g) was dissolved in a mixture of glycerol (6.30 g) and 1,2-propylene glycol (10.00 g) with the help of an ultra turrax at 7,000 rpm for 10 s. Perfume oil A (10.00 g) was dispersed in this solution with the help of an ultra turrax at 7,000 rpm for 2 min at 0°C. Succinyl chloride (15.20 g) was added to the first emulsion over the course of 1.25 h at room temperature. Then, adipoyl chloride (3.95 g) was added to this emulsion over the course of 1.25 h at room temperature. The reaction mixture was sitrred at room temperature for 4 h. The resulting emulsion was dispersed in a solution of PGPR90 (0.5g) (S2) in Neobee (25g) at 550 rpm with the help of a mechanical stirrer for 10 min at room temperature to afford a multiple emulsion. Adipoyl chloride (2 g) was added dropwise to the multiple emulsion over the course of 2 h, and the reaction mixture was stirred at 25°C for 24 h.

**Capsules 3F:** Hydroxypropyl cellulose (S1) (0.20 g) was dissolved in 1,2-propylene glycol (9.80 g). This solution was mixed with glycerol (6.30 g) with the help of an ultra turrax at 10,000 rpm for 10 s. Perfume oil A (10.00 g) was dispersed in this solution with the help of an ultra turrax at 10,000 rpm for 2 min. Succinyl chloride (15.15 g) was added to the first emulsion over the course of 1.25 h at room temperature. The reaction mixture was stirred at room temperature for 4 h. The resulting emulsion was dispersed in a solution of PGPR90 (0.5g) (S2) in Neobee (25g) at 550 rpm with the help of a mechanical stirrer for 10 min at room temperature to afford a multiple emulsion. Adipoyl chloride (2 g) was added dropwise to the multiple emulsion over the course of 2 h, and the reaction mixture was stirred at 25°C for 24 h.

**Capsules 3G:** Capsules 3G were prepared according to the protocol of capsules 3F. Perfume oil A (10.00 g) was dispersed in the polyol solution with the help of a mechanical stirrer equipped with an anchor stirred at 650 rpm for 10 min.

**Capsules 3H:** Hydroxypropylcellulose (S1) (0.20 g) was dissolved in a mixture of glycerol (12.60 g) and 1,2-propylene glycol (20.00 g) with the help of an ultra turrax at 7,000 rpm for 10 s. Perfume oil A (20.00 g) was dispersed in this solution with the help of an ultra turrax at 25,000 rpm for 2 min at 0°C. Succinyl chloride (30.00 g) was added to the first emulsion over the course of 1 h at room temperature. The reaction mixture was stirred at room temperature for 24 h. The resulting emulsion was dispersed in a solution of PGPR90 (S2) (1.00 g) in Neobee (49.00g) at 550 rpm with the help of a mechanical stirrer for 10 min at room temperature to afford a multiple emulsion. Succinyl chloride (10.00 g) was added dropwise to the multiple emulsion over the course of 1 h, and the reaction mixture was left at 25°C for 12 h.

**Capsules 4A:** Tween 20 (0.67 g) was dissolved in a mixture of glycerol (21.12 g) and 1,4-butanediol (33.60 g). Perfume oil A (22.80 g) was dispersed in this solution with the help of an ultra turrax at 24,000 rpm for 2 min. Succinyl chloride (14.40 g) was added to the first emulsion over the course of 1 h at room temperature. The reaction mixture was stirred at 40°C for 4 h. The resulting emulsion was dispersed in a solution of PGPR90 (1.00 g) in Neobee (50.00g) at 550 rpm with the help of a mechanical stirrer to afford a multiple emulsion. Succinyl chloride (10.00 g) was added dropwise to the multiple emulsion over the course of 1 h, and the reaction mixture was left at 25°C for 12 h.

### Example 4

### Fabric conditioner composition

Microcapsule slurry (see examples 1-3) are dispersed in a fabric conditioner base described in Table below to obtain a concentration of encapsulated perfume oil at 0.22%.

**Table 8 - Fabric conditioner composition**

| Product | Origin | Wt % |
|---|---|---|
| Stepantex VL 90A | | 8.88 |
| Calcium Chloride Sol. 10% | | 0.36 |
| Proxel GXL | Avecia | 0.04 |
| Perfume | Firmenich SA | 1 |
| Water | | 89.72 |
| TOTAL | | 100 |

### Example 5

### Liquid detergent composition

Microcapsule slurry (see examples 1-3) is dispersed in a liquid detergent base described in Table 9 to obtain a concentration of encapsulated perfume oil at 0.22%.

**Table 9 - Liquid detergent composition**

| Ingredients | Concentration [wt%] |
|---|---|
| Sodium C14-17 Alkyl Sec Sulfonate¹⁾ | 7 |
| Fatty acids, C12-18 and C18-unsaturated²⁾ | 7.5 |
| C12/14 fatty alcohol polyglycol ether with 7 mol EO³⁾ | 17 |
| Triethanolamine | 7.5 |
| Propylene Glycol | 11 |
| Citric acid | 6.5 |
| Potassium Hydroxyde | 9.5 |
| Protease | 0.2 |
| Amylase | 0.2 |
| Mannanase | 0.2 |
| Acrylates/Steareth-20 Methacrylate structuring Crosspolymer⁴⁾ | 6 |
| Deionized Water | 27.4 |

| | |
|---|---|
| 1) Hostapur SAS 60; Origin: Clariant 2) Edenor K 12-18; Origin: Cognis 3) Genapol LA 070; Origin: Clariant 4) Aculyn 88; Origin: Dow Chemical | |

### Example 6

### Rinse-off conditioner

Microcapsule slurry (see examples 1-3) is dispersed in a rinse-off conditioner base described in table 10 to obtain a concentration of encapsulated perfume oil at 0.5%.

**Table 10 - Rinse-off conditioner composition**

| | **Ingredients** | **Concentration [wt%]** |
|---|---|---|
| **A** | Water deionized | **81.8** |
| | Behentrimonium Chloride ¹⁾ | **2.5** |
| | Hydroxyethylcellulose ²⁾ | **1.5** |
| | | |
| **B** | Cetearyl Alcohol ³⁾ | **4** |
| | Glyceryl Stearate (and) PEG-100 Stearate ⁴⁾ | **2** |
| | Behentrimonium Methosulfate (and) Cetyl alcohol (and) Butylene Glycol ⁵⁾ | **4** |
| | Ethoxy (20) Stearyl Alcohol ⁶⁾ | **1** |
| | | |
| **C** | Amodimethicone (and) Trideceth-12 (and) Cetrimonium Chloride ⁷⁾ | **3** |
| | Chlorhexidine Digluconate ⁸⁾ 20% aqueous solution | **0.2** |
| **D** | Citric acid 10% aqueous sol. till pH 3.5-4 | **q.s.** |
| | **TOTAL:** | **100** |

| | | |
|---|---|---|
| 1) Genamin KDM P, Clariant 2) Tylose H10 Y G4, Shin Etsu 3) Lanette O, BASF 4) Arlacel 165-FP-MBAL-PA-(RB), Croda 5) Incroquat Behenyl TMS-50-MBAL-PA-(MH) HA4112, Croda 6) SP Brij S20 MBAL-PA(RB), Croda 7) Xiameter DC MEM-0949 Emulsion, Dow Corning 8) Alfa Aesar | | |

### Example 7

### Shampoo composition

Microcapsule slurry (see examples 1-3) is weighed and mixed in a shampoo composition to add the equivalent of 0.2% perfume.

**Table 11 - Shampoo composition**

| | **Ingredients** | **Concentration [wt%]** |
|---|---|---|
| **A** | Water deionized | **44.4** |
| | Polyquaternium-10 ¹⁾ | **0.3** |
| | Glycerin 85% ²⁾ | **1** |
| | DMDM Hydantoin ³⁾ | **0.2** |
| | | |
| **B** | Sodium Laureth Sulfate ⁴⁾ | **28** |
| | Cocamidopropyl Betaine ⁵⁾ | **3.2** |
| | Disodium Cocoamphodiacetate ⁶⁾ | **4** |
| | Ethoxy (20) Stearyl Alcohol ⁶⁾ | **1** |
| | | |
| **C** | Sodium Laureth Sulfate ⁴⁾ | **3** |
| | Glyceryl Laureate ⁷⁾ | **0.2** |
| | | |
| **D** | Water deionized | **1** |
| | Sodium Methylparaben ⁸⁾ | **0.1** |
| | | |
| **E** | Sodium Chloride 10% aqueous sol. | **15** |
| | Citric acid 10% aqueous sol. till pH 5.5-6 | **q.s.** |
| | Perfume | **0.5** |
| | **TOTAL:** | **100** |

| | | |
|---|---|---|
| 1) Ucare Polymer JR-400, Noveon 2) Schweizerhall 3) Glydant, Lonza 4) Texapon NSO IS, Cognis 5) Tego Betain F 50, Evonik 6) Amphotensid GB 2009, Zschimmer & Schwarz 7) Monomuls 90 L-12, Gruenau 8) Nipagin Monosodium, NIPA | | |

### Example 8

### Antiperspirant roll-on emulsion composition

Microcapsule slurry (see examples 1-3) is weighed and mixed in antiperspirant roll-on emulsion composition to add the equivalent of 0.2% perfume.

**Table 12 - Antiperspirant composition**

| **Ingredient** | **Amount (wt %)** |
|---|---|
| Steareth-2¹⁾ (Part A) | 3.25 |
| Steareth-21²⁾ (Part A) | 0.75 |
| PPG-15 Stearyl Ether³⁾ (Part A) | 4 |
| WATER deionised (Part B) | 51 |
| Aluminum Chlorohydrate 50% aqueous solution⁴⁾ (Part C) | 40 |
| Fragrance (Part D) | 1 |

| | |
|---|---|
| 1) BRIJ 72; origin : ICI 2) BRIJ 721; origin : ICI 3) ARLAMOL E; origin : UNIQEMA-CRODA 4) LOCRON L; origin : CLARIAN | |

Part A and B are heated separately to 75°C; Part A is added to Part B under stirring and the mixture is homogenized for 10 min. Then, the mixture is cooled under stirring; and Part C is slowly added when the mixture reached 45°C and Part D when the mixture reached at 35 °C while stirring. Then the mixture is cooled to room temperature.

### Example 9

### Shower-gel composition

Microcapsule slurry (see examples 1-3) is weighed and mixed in the following composition to add the equivalent of 0.2% perfume.

**Table 13 - Shower gel composition**

| **Ingredients** | **Amount (% wt)** | **Function** |
|---|---|---|
| WATER deionised | 49.350 | Solvent |
| Tetrasodium EDTA ¹⁾ | 0.050 | Chelating agent |
| Acrylates Copolymer²⁾ | 6.000 | Thickener |
| Sodium C12-C15 Pareth Sulfate ³⁾ | 35.000 | Surfactant |
| Sodium Hydroxide 20% aqueous solution | 1.000 | pH adjuster |
| Cocamidopropyl Betaine⁴⁾ | 8.000 | Surfactant |
| Methylchloroisothiazolinone and Methylisothiazolinone⁵⁾ | 0.100 | Preservative |
| Citric Acid (40%) | 0.500 | pH adjuster |

| | | |
|---|---|---|
| 12) EDETA B POWDER; trademark and origin: BASF 13) CARBOPOL AQUA SF-1 POLYMER; trademark and origin: NOVEON 14) ZETESOL AO 328 U; trademark and origin: ZSCHIMMER & SCHWARZ 15) TEGO-BETAIN F 50; trademark and origin: GOLDSCHMIDT 16) KATHON CG; trademark and origin: ROHM & HASS | | |

## Claims

1. Process for preparing a polyester microcapsule slurry, said process comprising the steps of:
(i) Dispersing an oil phase O1 comprising a hydrophobic material H1, into a continuous phase comprising a polyol to obtain a two-phases dispersion E1, wherein the oil phase O1 and/or the continuous phase comprise a stabilizer S1,
(ii) Dispersing the two-phases dispersion E1 in an oil phase O2 comprising a hydrophobic material H2 and a stabilizer S2 to obtain a multiple dispersion E2;
(iii) Adding at least one carboxylic acid derivative A3 into the multiple dispersion E2; and
(iv) applying conditions sufficient to induce interfacial polymerization and form polyester microcapsules in the form of a slurry,
wherein the carboxylic acid derivative A3 is chosen in the group consisting of acid, amide, ester, anhydride and acid chloride and mixtures thereof.

2. The process according to claim 1, wherein the hydrophobic material H1 comprises a perfume oil.

3. The process according to claim 1 or 2, wherein the process comprises a step v) consisting of adding a hydrophobic material H3, preferably a perfume oil, in the slurry.

4. The process according to claim 3, wherein the carboxylic acid derivative A3 is an acyl chloride.

5. The process according to claim 4, wherein the acyl chloride has the following formula (I) 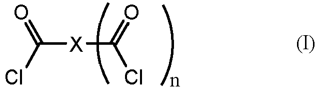
wherein n is an integer varying between 1 and 8, preferably between 1 and 6, more preferably between 1 and 4, and
wherein X is an (n+1)-valent C₂ to C₄₅ hydrocarbon group optionally comprising at least one group selected from (i) to (vi), 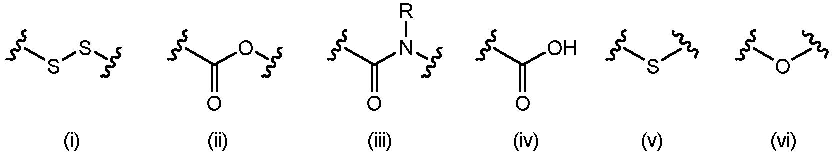
wherein R is a hydrogen atom or a methyl or ethyl group, preferably a hydrogen atom.

6. The process according to claim 5, wherein the acyl chloride is chosen from the group consisting of benzene-1,3,5-tricarbonyl trichloride (trimesoyl trichloride), benzene-1,2,4-tricarbonyl trichloride, benzene-1,2,4,5-tetracarbonyl tetrachloride, cyclohexane-1,3,5-tricarbonyl trichloride, isophthalyol dichloride, diglycolyl dichloride, terephthaloyl chloride, fumaryl dichloride, adipoyl dichloride, succinic acid dichloride, propane-1,2,3-tricarbonyl trichloride, cyclohexane-1,2,4,5-tetracarbonyl tetrachloride, 2,2'-disulfanediyldisuccinyl dichloride, 2-(2-chloro-2-oxo-ethyl)sulfanylbutanedioyl dichloride, (4-chloro-4-oxobutanoyl)-L-glutamoyl dichloride, (S)-4-((1,5-dichloro-1,5-dioxopentan-2-yl)amino)-4-oxobutanoic acid, 2,2-bis[(4-chloro-4-oxo-butanoyl)oxymethyl]butyl 4-chloro-4-oxo-butanoate, [2-[2,2-bis[(4-chloro-4-oxo-butanoyl)oxymethyl]butoxymethyl]-2-[(4-chloro-4-oxo-butanoyl)oxymethyl]butyl] 4-chloro-4-oxo-butanoate, 2,2-bis[(2-chlorocarbonylbenzoyl)oxymethyl]butyl 2-chlorocarbonyl-benzoate, [2-[2,2-bis[(2-chlorocarbonylbenzoyl)oxymethyl]butoxymethyl]-2-[(2-chlorocarbonylbenzoyl)oxymethyl]butyl] 2-chlorocarbonylbenzoate, 4-(2,4,5-trichlorocarbonylbenzoyl)oxybutyl 2,4,5-trichlorocarbonyl-benzoate, propane-1,2,3-triyl tris(4-chloro-4-oxobutanoate), propane-1,2-diyl bis(4-chloro-4-oxobutanoate) and mixtures thereof.

7. The process according to any one of the preceding claims, wherein at least one carboxylic acid derivative A1 is added into E1 after step i) and/or at least one carboxylic acid derivative A2 is added into the oil phase O1 and wherein the carboxylic acid derivative A1 and/or the carboxylic acid derivative A2 is preferably chosen in the group consisting of acid, amide, ester, anhydride and acid chloride and mixtures thereof, more preferably is an acid chloride, even more preferably is chosen in the group consisting of benzene-1,3,5-tricarbonyl trichloride (trimesoyl trichloride), benzene-1,2,4-tricarbonyl trichloride, benzene-1,2,4,5-tetracarbonyl tetrachloride, cyclohexane-1,3,5-tricarbonyl trichloride, isophthalyol dichloride, diglycolyl dichloride, terephthaloyl chloride, fumaryl dichloride, adipoyl dichloride, succinic dichloride, propane-1,2,3-tricarbonyl trichloride, cyclohexane-1,2,4,5-tetracarbonyl tetrachloride, 2,2'-disulfanediyldisuccinyl dichloride, 2-(2-chloro-2-oxoethyl)sulfanylbutanedioyl dichloride, (4-chloro-4-oxobutanoyl)-L-glutamoyl dichloride, (S)-4-((1,5-dichloro-1,5-dioxopentan-2-yl)amino)-4-oxobutanoic acid, 2,2-bis[(4-chloro-4-oxo-butanoyl)oxymethyl]butyl 4-chloro-4-oxo-butanoate, [2-[2,2-bis[(4-chloro-4-oxo-butanoyl)oxymethyl]butoxymethyl]-2-[(4-chloro-4-oxo-butanoyl)oxymethyl]butyl] 4-chloro-4-oxo-butanoate, 2,2-bis[(2-chlorocarbonylbenzoyl)oxymethyl]butyl 2-chlorocarbonyl-benzoate, [2-[2,2-bis[(2-chlorocarbonylbenzoyl)oxymethyl]butoxymethyl]-2-[(2-chlorocarbonylbenzoyl)oxymethyl]butyl] 2-chlorocarbonylbenzoate, 4-(2,4,5-trichlorocarbonylbenzoyl)oxybutyl 2,4,5-trichlorocarbonyl-benzoate, propane-1,2,3-triyl tris(4-chloro-4-oxobutanoate), propane-1,2-diyl bis(4-chloro-4-oxobutanoate) and mixtures thereof.

8. The process according to anyone of the preceding claims, wherein the continuous phase does not comprise water.

9. The process according to anyone of the preceding claims, wherein the polyol is chosen in the group consisting of glycerol,1,4-butanediol, ethylene glycol, 1,2-propylene glycol, 1,3-propylene glycol, triethanolamine, di(trimethylolpropane), ethylene glycol, glycerol, 1,4-butanediol, 1,2-hexanediol, 1,6-hexanediol, 2-ethyl-2-(hydroxymethyl)propane-1,3-diol (trimethylolpropane, TMP), 2,2-bis(hydroxymethyl)propane-1,3-diol (pentaerythritol), 2-amino-2-ethylpropane-1,3-diol, 2-amino-2-(hydroxymethyl)propane-1,3-diol, 2,2'-azanediylbis(ethan-1-ol), 2-aminopropane-1,3-diol, 2-amino-2-methylpropane-1,3-diol, polyphenols and mixtures thereof.

10. The process according to any one of the preceding claims, wherein the hydrophobic material H2 is chosen in the group consisting of triglycerides, isopropyl myristate, and mixtures thereof.

11. The process according to any one of the preceding claims, wherein the stabilizers S1 and/or S2 are is chosen in the group consisting secondary alcohol ethoxylate, polyethylene glycol sorbitan monooleate, hydrophobized silica particles; silk protein; sorbitan stearate, hydroxyapatite particles, polyglycerol polyricinoleate, sorbitane trioleate, polyglyceryl-3 diisostearate, cellulose derivatives such as hydroxypropylcellulose and mixtures thereof.

12. A polyester microcapsule slurry obtainable by the process as described in any one of the preceding claims, wherein it comprises at least
- a core comprising an oil phase, wherein the oil phase comprises a hydrophobic material H1, and
- a carrier comprising the reaction product between at least one carboxylic acid derivative A3 and a polyol,
wherein the core is entrapped in the carrier, and wherein the core comprises a two-phases dispersion made of the polyol and the hydrophobic material H1.

13. A consumer product comprising:
- a personal care active base, and
- microcapsules as defined in claim 12,
wherein the consumer product is in the form of a personal care composition.

14. A consumer product comprising:
- a home care or a fabric care active base, and
- microcapsules as defined in claim 12,
wherein the consumer product is in the form of a home care or a fabric care composition.

## Patentansprüche

1. Verfahren zur Herstellung einer Polyestermikrokapselaufschlämmung, wobei das Verfahren die folgenden Schritte umfasst:
(i) Dispergieren einer Ölphase O1, die ein hydrophobes Material H1 umfasst, in einer kontinuierlichen Phase, die ein Polyol umfasst, unter Erhalt einer zweiphasigen Dispersion E1, wobei die Ölphase O1 und/oder die kontinuierliche Phase einen Stabilisator S1 umfassen,
(ii) Dispergieren der zweiphasigen Dispersion E1 in einer Ölphase O2, die ein hydrophobes Material H2 und einen Stabilisator S2 umfasst, unter Erhalt einer multiplen Dispersion E2;
(iii) Zugeben mindestens eines Carbonsäurederivats A3 zu der multiplen Dispersion E2; und
(iv) Anwenden von Bedingungen, die ausreichen, um Grenzflächenpolymerisation zu induzieren und Polyestermikrokapseln in Form einer Aufschlämmung zu bilden,
wobei das Carbonsäurederivat A3 aus der Gruppe bestehend aus Säure, Amid, Ester, Anhydrid und Säurechlorid und Mischungen davon ausgewählt wird.

2. Verfahren nach Anspruch 1, wobei das hydrophobe Material H1 ein Parfümöl umfasst.

3. Verfahren nach Anspruch 1 oder 2, wobei das Verfahren einen Schritt v) umfasst, der aus dem Zugeben eines hydrophoben Materials H3, vorzugsweise eines Parfümöls, zu der Aufschlämmung besteht.

4. Verfahren nach Anspruch 3, wobei es sich bei dem Carbonsäurederivat A3 um ein Acylchlorid handelt.

5. Verfahren nach Anspruch 4, wobei das Acylchlorid die folgende Formel (I) aufweist: 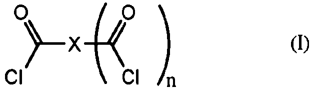
wobei n für eine ganze Zahl steht, die zwischen 1 und 8, vorzugsweise zwischen 1 und 6, weiter bevorzugt zwischen 1 und 4, variiert und
wobei X für eine (n+1)-wertige C₂-C₄₅-Kohlenwasserstoffgruppe steht, die gegebenenfalls mindestens eine Gruppe umfasst, die aus (i) bis (vi) ausgewählt ist, 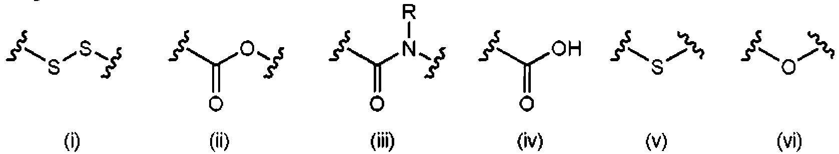
wobei R für ein Wasserstoffatom oder eine Methyl- oder Ethylgruppe, vorzugsweise ein Wasserstoffatom, steht.

6. Verfahren nach Anspruch 5, wobei das Acylchlorid aus der Gruppe bestehend aus Benzol-1,3,5-tricarbonyltrichlorid (Trimesoyltrichlorid), Benzol-1,2,4-tricarbonyltrichlorid, Benzol-1,2,4,5-tetracarbonyltetrachlorid, Cyclohexan-1,3,5-tricarbonyltrichlorid, Isophthalyoldichlorid, Diglykolyldichlorid, Terephthaloylchlorid, Fumaryldichlorid, Adipoyldichlorid, Bernsteinsäuredichlorid, Propan-1,2,3-tricarbonyltrichlorid, Cyclohexan-1,2,4,5-tetracarbonyltetrachlorid, 2,2'-Disulfandiyldisuccinyldichlorid, 2-(2-Chlor-2-oxoethyl)sulfanylbutandiyldichlorid, (4-Chlor-4-oxobutanoyl)-L-glutamoyldichlorid, (S)-4-((1,5-Dichlor-1,5-dioxopentan-2-yl)amino)-4-oxobutansäure, 4-Chlor-4-oxobutansäure-2,2-bis[(4-chlor-4-oxobutanoyl)oxymethyl]butylester, 4-Chlor-4-oxobutansäure[2-[2,2-bis[(4-chlor-4-oxobutanoyl)oxymethyl]butoxymethyl]-2-[(4-chlor-4-oxo-butanoyl)oxymethyl]butyl]ester, 2-Chlorcarbonylbenzoesäure-2,2-bis[(2-chlorcarbonylbenzoyl)oxymethyl]butylester, 2-Chlorcarbonylbenzoesäure[2-[2,2-bis[(2-chlorcarbonylbenzoyl)oxymethyl]butoxymethyl]-2-[(2-chlorcarbonylbenzoyl)oxymethyl]butyl]ester, 2,4,5-Trichlorcarbonylbenzoesäure-4-(2,4,5-trichlorcarbonylbenzoyl)oxybutylester, Propan-1,2,3-triyltris(4-chlor-4-oxobutanoat), Propan-1,2-diylbis(4-chlor-4-oxobutanoat) und Mischungen davon ausgewählt wird.

7. Verfahren nach einem der vorhergehenden Ansprüche, wobei mindestens ein Carbonsäurederivat A1 nach Schritt i) zu E1 gegeben wird und/oder mindestens ein Carbonsäurederivat A2 zu der Ölphase O1 gegeben wird und wobei das Carbonsäurederivat A1 und/oder das Carbonsäurederivat A2 vorzugsweise aus der Gruppe bestehend aus Säure, Amid, Ester, Anhydrid und Säurechlorid und Mischungen davon ausgewählt wird, weiter bevorzugt ein Säurechlorid ist und noch weiter bevorzugt aus der Gruppe bestehend aus Benzol-1,3,5-tricarbonyltrichlorid (Trimesoyltrichlorid), Benzol-1,2,4-tricarbonyltrichlorid, Benzol-1,2,4,5-tetracarbonyltetrachlorid, Cyclohexan-1,3,5-tricarbonyltrichlorid, Isophthalyoldichlorid, Diglykolyldichlorid, Terephthaloylchlorid, Fumaryldichlorid, Adipoyldichlorid, Bernsteinsäuredichlorid, Propan-1,2,3-tricarbonyltrichlorid, Cyclohexan-1,2,4,5-tetracarbonyltetrachlorid, 2,2'-Disulfandiyldisuccinyldichlorid, 2-(2-Chlor-2-oxoethyl)sulfanylbutandiyldichlorid, (4-Chlor-4-oxobutanoyl)-L-glutamoyldichlorid, (S)-4-((1,5-Dichlor-1,5-dioxopentan-2-yl)amino)-4-oxobutansäure, 4-Chlor-4-oxobutansäure-2,2-bis[(4-chlor-4-oxobutanoyl)oxymethyl]butylester, 4-Chlor-4-oxobutansäure[2-[2,2-bis[(4-chlor-4-oxobutanoyl)oxymethyl]butoxymethyl]-2-[(4-chlor-4-oxo-butanoyl)oxymethyl]butyl]ester, 2-Chlorcarbonylbenzoesäure-2,2-bis[(2-chlorcarbonylbenzoyl)oxymethyl]butylester, 2-Chlorcarbonylbenzoesäure[2-[2,2-bis[(2-chlorcarbonylbenzoyl)oxymethyl]butoxymethyl]-2-[(2-chlorcarbonylbenzoyl)oxymethyl]butyl]ester, 2,4,5-Trichlorcarbonylbenzoesäure-4-(2,4,5-trichlorcarbonylbenzoyl)oxybutylester, Propan-1,2,3-triyltris(4-chlor-4-oxobutanoat), Propan-1,2-diylbis(4-chlor-4-oxobutanoat) und Mischungen davon ausgewählt wird.

8. Verfahren nach einem der vorhergehenden Ansprüche, wobei die kontinuierliche Phase kein Wasser umfasst.

9. Verfahren nach einem der vorhergehenden Ansprüche, wobei das Polyol aus der Gruppe bestehend aus Glycerin, 1,4-Butandiol, Ethylenglykol, 1,2-Propylenglykol, 1,3-Propylenglykol, Triethanolamin, Di(trimethylolpropan), Ethylenglykol, Glycerin, 1,4-Butandiol, 1,2-Hexandiol, 1,6-Hexandiol, 2-Ethyl-2-(hydroxymethyl)propan-1,3-diol (Trimethylolpropan, TMP), 2,2-Bis(hydroxymethyl)propan-1,3-diol (Pentaerythrit), 2-Amino-2-ethylpropan-1,3-diol, 2-Amino-2-(hydroxymethyl)propan-1,3-diol, 2,2'-Azandiylbis(ethan-1-ol), 2-Aminopropan-1,3-diol, 2-Amino-2-methylpropan-1,3-diol, Polyphenolen und Mischungen davon ausgewählt wird.

10. Verfahren nach einem der vorhergehenden Ansprüche, wobei das hydrophobe Material H2 aus der Gruppe bestehend aus Triglyceriden, Isopropylmyristat und Mischungen davon ausgewählt wird.

11. Verfahren nach einem der vorhergehenden Ansprüche, wobei die Stabilisatoren S1 und/oder S2 aus der Gruppe bestehend aus sekundärem Alkoholethoxylat, Polyethylenglykolsorbitanmonooleat, hydrophobierten Siliciumdioxidpartikeln; Seidenprotein; Sorbitanstearat, Hydroxylapatitpartikeln, Polyglycerinpolyricinoleat, Sorbitantrioleat, Polyglyceryl-3-diisostearat, Cellulosederivaten wie Hydroxypropylcellulose und Mischungen davon ausgewählt werden.

12. Polyestermikrokapselaufschlämmung, erhältlich durch das in einem der vorhergehenden Ansprüche beschriebene Verfahren, wobei sie mindestens Folgendes umfasst:
- einen Kern, der eine Ölphase umfasst, wobei die Ölphase ein hydrophobes Material H1 umfasst, und
- einen Träger, der das Reaktionsprodukt zwischen mindestens einem Carbonsäurederivat A3 und einem Polyol umfasst,
wobei der Kern in dem Träger eingeschlossen ist und wobei der Kern eine zweiphasige Dispersion aus dem Polyol und dem hydrophoben Material H1 umfasst.

13. Verbraucherprodukt, umfassend:
- eine Körperpflegewirkstoffbasis und
- Mikrokapseln gemäß Anspruch 12,
wobei das Verbraucherprodukt in Form einer Körperpflegezusammensetzung vorliegt.

14. Verbraucherprodukt, umfassend:
- eine Haushaltspflege- oder Textilpflegewirkstoffbasis und
- Mikrokapseln gemäß Anspruch 12,
wobei das Verbraucherprodukt in Form einer Haushaltspflege- oder Textilpflegezusammensetzung vorliegt.

## Revendications

1. Procédé de préparation d'une suspension de microcapsules de polyester, ledit procédé comprenant les étapes de :
(i) dispersion d'une phase huileuse O1 comprenant un matériau hydrophobe H1, dans une phase continue comprenant un polyol pour obtenir une dispersion à deux phases E1, dans laquelle la phase huileuse O1 et/ou la phase continue comprennent un stabilisant S1,
(ii) dispersion de la dispersion à deux phases E1 dans une phase huileuse O2 comprenant un matériau hydrophobe H2 et un stabilisant S2 pour obtenir une dispersion multiple E2 ;
(iii) ajout d'au moins un dérivé d'acide carboxylique A3 dans la dispersion multiple E2 ; et
(iv) application de conditions suffisantes pour induire une polymérisation interfaciale et former des microcapsules de polyester sous la forme d'une suspension,
dans lequel le dérivé d'acide carboxylique A3 est choisi dans le groupe constitué par un acide, amide, ester, anhydride, chlorure d'acide et leurs mélanges.

2. Procédé selon la revendication 1, dans lequel le matériau hydrophobe H1 comprend une huile de parfum.

3. Procédé selon la revendication 1 ou 2, dans lequel le procédé comprend une étape v) consistant à ajouter un matériau hydrophobe H3, préférablement une huile de parfum, dans la suspension.

4. Procédé selon la revendication 3, dans lequel le dérivé d'acide carboxylique A3 est un chlorure d'acyle.

5. Procédé selon la revendication 4, dans lequel le chlorure d'acyle présente la formule (I) suivante 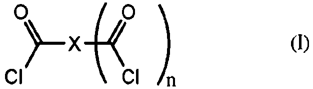
dans laquelle n est un entier variant entre 1 et 8, préférablement entre 1 et 6, plus préférablement entre 1 et 4, et
dans laquelle X est un groupe hydrocarboné en C₂ à C₄₅ de valence (n+1) comprenant éventuellement au moins un groupe choisi parmi (i) à (vi), 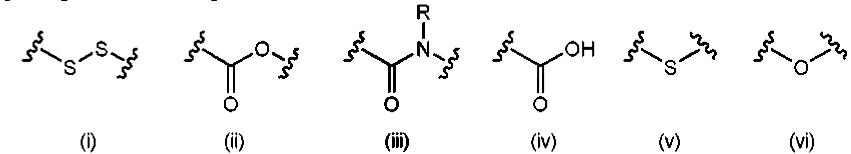
dans laquelle R est un atome d'hydrogène ou un groupe méthyle ou éthyle, préférablement un atome d'hydrogène.

6. Procédé selon la revendication 5, dans lequel le chlorure d'acyle est choisi dans le groupe constitué par trichlorure de benzène-1,3,5-tricarbonyle (trichlorure de trimésoyle), trichlorure de benzène-1,2,4-tricarbonyle, tétrachlorure de benzène-1,2,4,5-tétracarbonyle, trichlorure de cyclohexane-1,3,5-tricarbonyle, dichlorure d'isophtaloyle, dichlorure de diglycolyle, chlorure de téréphtaloyle, dichlorure de fumaryle, dichlorure d'adipoyle, dichlorure d'acide succinique, trichlorure de propane-1,2,3-tricarbonyle, tétrachlorure de cyclohexane-1,2,4,5-tétracarbonyle, dichlorure de 2,2'-disulfanediyldisuccinyle, dichlorure de 2-(2-chloro-2-oxo-éthyl)sulfanylbutanedioyle, dichlorure de (4-chloro-4-oxobutanoyl)-L-glutamoyle, acide (S)-4-((1,5-dichloro-1,5-dioxopentan-2-yl)amino)-4-oxobutanoïque, 4-chloro-4-oxo-butanoate de 2,2-bis[(4-chloro-4-oxo-butanoyl)oxyméthyl]butyle, 4-chloro-4-oxo-butanoate de [2-[2,2-bis[(4-chloro-4-oxo-butanoyl)oxyméthyl]butoxyméthyl]-2-[(4-chloro-4-oxo-butanoyl)oxyméthyl]butyle], 2-chlorocarbonyl-benzoate de 2,2-bis[(2-chlorocarbonylbenzoyl)oxyméthyl]butyle, 2-chlorocarbonylbenzoate de [2-[2,2-bis[(2-chlorocarbonylbenzoyl)oxyméthyl]butoxyméthyl]-2-[(2-chlorocarbonylbenzoyl)oxyméthyl]butyle], 2,4,5-trichlorocarbonyl-benzoate de 4-(2,4,5-trichlorocarbonylbenzoyl)oxybutyle, tris(4-chloro-4-oxobutanoate) de propane-1,2,3-triyle, bis(4-chloro-4-oxobutanoate) de propane-1,2-diyle et leurs mélanges.

7. Procédé selon l'une quelconque des revendications précédentes, dans lequel au moins un dérivé d'acide carboxylique A1 est ajouté dans E1 après l'étape i) et/ou au moins un dérivé d'acide carboxylique A2 est ajouté dans la phase huileuse 01 et dans lequel le dérivé d'acide carboxylique A1 et/ou le dérivé d'acide carboxylique A2 préférablement est choisi dans le groupe constitué par un acide, un amide, un ester, un anhydride et un chlorure d'acide et des mélanges de ceux-ci, plus préférablement est un chlorure d'acide, encore plus préférablement est choisi dans le groupe constitué par trichlorure de benzène-1,3,5-tricarbonyle (trichlorure de trimésoyle), trichlorure de benzène-1,2,4-tricarbonyle, tétrachlorure de benzène-1,2,4,5-tétracarbonyle, trichlorure de cyclohexane-1,3,5-tricarbonyle, dichlorure d'isophtaloyle, dichlorure de diglycolyle, chlorure de téréphtaloyle, dichlorure de fumaryle, dichlorure d'adipoyle, dichlorure d'acide succinique, trichlorure de propane-1,2,3-tricarbonyle, tétrachlorure de cyclohexane-1,2,4,5-tétracarbonyle, dichlorure de 2,2'-disulfanediyldisuccinyle, dichlorure de 2-(2-chloro-2-oxo-éthyl)sulfanylbutanedioyle, dichlorure de (4-chloro-4-oxobutanoyl)-L-glutamoyle, acide (S)-4-((1,5-dichloro-1,5-dioxopentan-2-yl)amino)-4-oxobutanoïque, 4-chloro-4-oxo-butanoate de 2,2-bis[(4-chloro-4-oxo-butanoyl)oxyméthyl]butyle, 4-chloro-4-oxo-butanoate de [2-[2,2-bis[(4-chloro-4-oxo-butanoyl)oxyméthyl]butoxyméthyl]-2-[(4-chloro-4-oxo-butanoyl)oxyméthyl]butyle], 2-chlorocarbonyl-benzoate de 2,2-bis[(2-chlorocarbonylbenzoyl)oxyméthyl]butyle, 2-chlorocarbonylbenzoate de [2-[2,2-bis[(2-chlorocarbonylbenzoyl)oxyméthyl]butoxyméthyl]-2-[(2-chlorocarbonylbenzoyl)oxyméthyl]butyle], 2,4,5-trichlorocarbonyl-benzoate de 4-(2,4,5-trichlorocarbonylbenzoyl)oxybutyle, tris(4-chloro-4-oxobutanoate) de propane-1,2,3-triyle, bis(4-chloro-4-oxobutanoate) de propane-1,2-diyle et leurs mélanges.

8. Procédé selon l'une quelconque des revendications précédentes, dans lequel la phase continue ne comprend pas d'eau.

9. Procédé selon l'une quelconque des revendications précédentes, dans lequel le polyol est choisi dans le groupe constitué par glycérol, 1,4-butanediol, éthylène glycol, 1,2-propylène glycol, 1,3-propylène glycol, triéthanolamine, di(triméthylolpropane), éthylène glycol, glycérol, 1,4-butanediol, 1,2-hexanediol, 1,6-hexanediol, 2-éthyl-2-(hydroxyméthyl)propane-1,3-diol (triméthylolpropane, TMP), 2,2-bis(hydroxyméthyl)propane-1,3-diol (pentaérythritol), 2-amino-2-éthylpropane-1,3-diol, 2-amino-2-(hydroxyméthyl)propane-1,3-diol, 2,2'-azanediylbis(éthan-1-ol), 2-aminopropane-1,3-diol, 2-amino-2-méthylpropane-1,3-diol, polyphénols et leurs mélanges.

10. Procédé selon l'une quelconque des revendications précédentes, dans lequel le matériau hydrophobe H2 est choisi dans le groupe constitué par des triglycérides, le myristate d'isopropyle et leurs mélanges.

11. Procédé selon l'une quelconque des revendications précédentes, dans lequel les stabilisants S1 et/ou S2 sont choisis dans le groupe constitué par un éthoxylate d'alcool secondaire, un monooléate de sorbitane de polyéthylène glycol, des particules de silice hydrophobisées ; une protéine de soie ; le stéarate de sorbitane, des particules d'hydroxyapatite, un polyricinoléate de polyglycérol, le trioléate de sorbitane, un diisostéarate de polyglycéryle-3, des dérivés de cellulose tels qu'une hydroxypropylcellulose, et leurs mélanges.

12. Suspension de microcapsules de polyester pouvant être obtenue par le procédé tel que décrit dans l'une quelconque des revendications précédentes, dans laquelle elle comprend au moins
- un noyau comprenant une phase huileuse, dans lequel la phase huileuse comprend un matériau hydrophobe H1, et
- un support comprenant le produit de réaction entre au moins un dérivé d'acide carboxylique A3 et un polyol,
dans laquelle le noyau est piégé dans le support, et dans laquelle le noyau comprend une dispersion à deux phases composée du polyol et du matériau hydrophobe H1.

13. Produit de consommation comprenant :
- une base active de soins personnels, et
- des microcapsules telles que définies dans la revendication 12,
dans lequel le produit de consommation se présente sous la forme d'une composition de soins personnels.

14. Produit de consommation comprenant :
- une base active d'entretien ménager ou d'entretien de tissus, et
- des microcapsules telles que définies dans la revendication 12,
le produit de consommation étant sous la forme d'une composition d'entretien ménager ou d'entretien de tissus.
